(19) Europäisches Patentamt
European Patent Office
Office européen des brevets

(11) Publication number: **0 153 188 B1**

(12) **EUROPEAN PATENT SPECIFICATION**

(45) Date of publication of patent specification: **09.10.91**

(21) Application number: **85301110.4**

(22) Date of filing: **19.02.85**

(51) Int. Cl.⁵: **C12N 15/30**, C12N 15/73, C07K 15/00, C12P 21/02, C12P 21/08, A61K 39/015, C12P 21/00, C12N 1/20, C12N 5/00, C07K 17/00, //(C12P21/00,C12R1:91), (C12N1/20,C12R1:19)

(54) Improvements in or relating to the production of malaria vaccines.

(30) Priority: 21.02.84 GB 8404493
13.11.84 GB 8428643
21.12.84 GB 8432337

(43) Date of publication of application:
28.08.85 Bulletin 85/35

(45) Publication of the grant of the patent:
09.10.91 Bulletin 91/41

(84) Designated Contracting States:
AT BE CH DE FR GB IT LI NL

(56) References cited:
WO-A-84/02917
WO-A-84/02922
GB-A- 2 096 893
GB-A- 2 099 300

(73) Proprietor: **NATIONAL RESEARCH DEVELOP-MENT CORPORATION**
**101 Newington Causeway**
**London SE1 6BU(GB)**

(72) Inventor: **Hope, Ian Allen**
**21 Musgrave Road**
**Chinnor Oxford OX9 4PL(GB)**
Inventor: **Scaife, John Graham**
**18A Inverleith Row**
**Edinburgh EH3 5LS Scotland(GB)**
Inventor: **Strambachova-McBride, Jana**
**153 Warrender Park Road**
**Edinburgh EH9 1DT Scotland(GB)**

EP 0 153 188 B1

NATURE, vol. 36, 22/29-12-1983, Macmillan Journals Ltd., pp. 751-754; R. L. Coppel et al, "Isolate specific S-antigen of Plasmodium falciparum contains a repeated sequence of eleven amino acids"

NATURE, vol.305, September 1983, no. 5929, Bucks, GB, pp. 29-33, Macmillan Journals Ltd., GB; G. N. Godson et al, "Identification and chemical synthesis of a tandemly repeated immunogenic region of Plasmodium knowlesi cicumsporozoite protein"

PROCEEDINGS OF THE NATIONAL ACADEMY OF SCIENCES, USA, vol. 80, pp. 3787-3791, June 1983, Immunology; D.J. Kemp et al, "Expression of Plasmodium falciparum blood-stage antigens in Escherichia coli: Detection with antibodies from immune humans"

CHEMICAL ABSTRACTS, vol.97, no. 9, 30-8-1982, p. 152, ref. 67168g, Columbus, Ohio, US; M. Goman et al, "The establishment of genomic DNA libraries for the human malaria parasite Plasmodium falciparum and identification of individual clones by hybridization"

BIOLOGICAL ABSTRACTS/RRM, Biosciences Information Service, ref. 19103, Philadelphia, US; J. Scaife et al, "Application of recombinant DNA techniques to the human malaria parasite Plasmodium falciparum. 12th annual UCLA Symposium on molecular biology of Host-Parasite interactions

NATURE, vol. 308, 08-03-1984, Macmillan Journals Ltd., pp. 191-194; I.A. Hope et al, "Evidence for immunological cross-reaction between sporozoites and blood stages of a human malaria parasite"

NUCLEIC ACIDS RESEARCH, vol. 13, January 1985, pp. 369-379, no. 2, Oxford, GB; I. A. Hope et al, "The gene for an exported antigen of the malaria parasite Plasmodium falciparum cloned and expressed in Escherichia coli"

(74) Representative: Stephenson, Gerald Frederick Patent Department National Research Development Corporation 101 Newington Causeway
London SE1 6BUGB

2

## Description

Malaria parasites (Plasmodium spp.) show a complex pattern of development in the mammalian host. Infection is initiated when female mosquitoes feeding on blood inoculate sporozoites into the blood stream from whence they enter the liver. The parasites proliferate in the liver and are ultimately released therefrom in the mature form, known as merozoites, which initiate the asexual erythrocytic cycle which causes the disease. The parasites, once taken up as merozoites by the red blood cells, pass through various stages, including the trophozoite and schizont stages, which culminate in the rupture of the blood cells with the release of much increased number of merozoites, thereby perpetuating the cycle.

Malaria presents a very considerable medical problem, despite the development of various drugs for its treatment. This is particularly the case since, of the four species of Plasmodium responsible for malaria in humans, P. falciparum is the species which causes the most morbidity and mortality and is at the same time the one which poses most problems by way of resistance to the preferred therapeutic agent, chloroquine. Much work has been directed towards the development of malaria vaccines but this has so far met with little success, due largely to the complex life cycle of the parasite, involving both sporozoite and various erythrocytic stages

It is a commonly held view expressed in a recent authoritative review (Cox, Nature, 1983, 304, 13) that the sporozoite surface and the intra-erythrocytic stages of the malaria parasite are immunologically distinct and that any effective vaccine will necessarily require to be based on a complex cocktail of antigens. The production of suitable antigenic materials capable of providing antibodies against the sporozoite stage of the parasite has presented particular problems.

We have now been able to show that this commonly held view is incorrect and that the sporozoite surface and the intra-erythrocytic stages of the parasite do possess antigens which will react with a common antibody, this finding being of particular importance since it means that antigenic materials can be prepared which will generate antibodies reactive against two markedly different stages of the parasite's life cycle. Such antigenic materials may advantageously be based upon antigens present in the intra-erythrocytic stages of the parasite thereby providing a high level of efficacy in vaccination against intra-erythrocytic forms of the parasite, whilst at the same time inducing antibodies active against the sporozoite surface.

Accordingly the present invention comprises an antigenic material suitable for use in vaccination against malaria which (a) is an antigenic substance characterised by:

(1) being an immunogenic proteinaceous antigen of a parasite of the species Plasmodium falciparum occurring in nature in association with intra-erythrocytic forms of the parasite;

(2) having a molecular weight as determined by SDS-PAGE in the range of 20,000 to 25,000 daltons; and

(3) comprising a sequence of amino acid residues containing an epitope, which epitope is shared with an antigenic material located on the sporozoite stage of the parasite so that on administration in vivo the antigenic substance generates antibodies reactive with both intra-erythrocytic forms of the parasite and with the sporozoite surface of that parasite;

or (b) is an antigenic substance derivable from the antigenic substance defined in (a) and which contains a whole or a part of the sequence of amino acid residues therein including those residues which constitute said epitope so that on administration in vivo the antigenic substance generates antibodies reactive with both intra-erythrocytic forms of the parasite and with the sporozoite surface of that parasite.

It will be appreciated that although antigenic materials according to the present invention are characterised by this one particular form of epitope they may however also contain other epitopes in addition.

The epitope occurring in nature in association with intra-erythrocytic forms of the parasite is shared with the surface of sporozoites since both the surface of the sporozoites and the intra-erythrocytic stages of the parasite carry an antigen containing an epitope which is reactive with a common antibody site, or paratope. Howver, this does not necessarily mean that the epitope, in terms of the sequence of amino acid residues by which it is constituted, will be present in identical form on the sporozoite surface and in association with intra-erythrocytic forms of the parasite, but rather that the particular amino acid residues which are present in the epitope will in each case have a specific reactivity for the common antibody site. The intra-erythrocytic stage epitope and the sporozoite epitope will thus share the same activity, even though they may not be completely identical. Moreover, it will be appreciated that the amino acid residues constituting the epitope may be in consecutive or non-consecutive sequence.

Whilst the detailed structure of the epitope may vary between different strains of P. falciparum, it is believed that the naturally occurring intra-erythrocytic stage antigens containing such epitopes constitute a quite closely similar group of materials, each possessing a molecular weight within the range of 20,000 to 25,000 daltons. The molecular weights used herein, unless otherwise specifically indicated, are those obtained by the sodium dodecyl sulphate-polyacrylamide gel electrophoresis (SDS-PAGE) procedure utilising standard molecular weight markers and as described by Laemmli, Nature, 1970, 227, 680.

It will be appreciated, however, that the present invention is not limited to such antigens as occur in nature, whether isolated therefrom or synthetically produced, but also extends to artificially produced materials of a molecular weight in the range of 20,000 to 25,000 daltons containing an epitope which occurs in one of the natural antigens of P. falciparum, such artificially produced materials being derived from the natural antigens or produced synthetically. Moreover, as indicated, the invention also extends to antigenic substances containing such an epitope derivable from the antigenic substance of molecular weight 20,000 to 25,000 daltons. However, for reasons discussed hereinafter, both naturally derived and synthetic materials with a molecular weight in this range, for example 22,000 to 24,000 daltons, can have certain advantages and are therefore of particular interest.

As will be appreciated from the foregoing description, more effective control of malaria produced by the species P. falciparum is an aim of particular interest and a particularly important feature of the present invention derives from the identification of an epitope associated with the parasites in certain intra-erythrocytic stages of many strains of P. falciparum and which has a common reactivity with an epitope occurring on the surface of such P. falciparum sporozoites. We have designated this intra-erythrocytic stage epitope by the number 5.1-1 and, for simplicity, this designation is used hereinafter at various points in the specification. This epitope was identified by the use of a monoclonal antibody raised against P. falciparum parasites which, in contrast to any of the other monoclonal antibodies to the intra-erythrocytic stages of malaria parasites so far prepared, has the ability to bind to both the surface of sporozoites and to intra-erythrocytic stages of the parasite. The techniques involved in the preparation of a hybridoma which secretes a monoclonal antibody of such specificity are the conventional techniques of the hybridoma art which are by now well known. The literature contains reports of the use of these conventional techniques to prepare monoclonal antibodies reactive with a variety of malaria antigens. Indeed, two papers, McBride et al, Science, 1982, 217, 255 and Hall et al, Molecular and Biochemical Parasitology, 1983, 7, 247, refer to a range of such monoclonal antibodies reactive with intra-erythrocytic stage antigens, including antibodies produced by a hybridoma identified as 5.1 which is closely related to the hybridoma 5.1-1 referred to hereinafter.

It will be seen, however, that the characterisation of the antibodies produced by the 5.1 hybridoma which is presented in these two papers fails to make any reference to their ability to react not only with an intra-erythrocytic stage antigen but also with sporozoites. Moreover the SDS-PAGE analysis of the $^{125}$I-labelled proteins with which the 5.1 hybridoma antibodies react presented in the Hall et al paper, which reports immunoprecipitation data, is erroneous in that it identifies these antibodies as reacting reproducibly with proteins having a molecular weight of 160, 60 and 35 k.daltons and sometimes with a protein having a molecular weight of 93 k.daltons. In contrast, the more specific and reproducible Western blotting data presented in Example 7 shows that the 5.1-1 epitope-specific antibody is characterised by reaction with a natural protein having a molecular weight on SDS-PAGE of 23 k.daltons. It will be appreciated, therefore, that the information provided in these publications is quite insufficient to enable one to repeat the preparation of the hybridoma 5.1 mentioned therein. Moreover, it should be emphasised that there is no suggestion in these papers that the 5.1 hybridoma referred to therein produces antibodies having a reactivity which is of any particular interest.

The present invention derives from the hitherto quite unexpected finding that monoclonal antibodies could be raised which are reactive not only with an epitope present in an intra-erythrocytic stage antigen of P. falciparum parasites but also with an epitope present on the surface of the corresponding P. falciparum sporozoites. Once the existence of such intra-erythrocytic stage epitopes has been identified and exemplified in the case of the P. falciparum epitope 5.1-1, the existing techniques of the art may be used to produce various hybridomas secreting monoclonal antibodies directed against such epitopes. In particular, although we have utilised one hybridoma, designated 5.1-1, producing monoclonal antibodies reactive against the 5.1-1 epitope, a wide range of hybridomas producing monoclonal antibodies of similar reactivity may of course be produced by conventional techniques once the existence of the 5.1-1 epitope has been identified.

The procedure used to produce the hybridoma cell line 5.1-1, and which may be utilised to produce alternative hybridomas secreting monoclonal antibodies reactive with the 5.1-1 epitope, involved the fusion between spleen cells from a Balb/c mouse immunized with P. falciparum parasites obtained from blood and NS-1/1-Ag4-1 (NS-1) myeloma cells (Kohler et al, European Journal of Immunology, 1976, 6, 292). The procedure is described hereinafter in Example 1 in full detail. Similarity of epitopic specificity for antibodies produced by alternative hybridomas to the 5.1-1 hybridoma may be determined through their reactivity with both sporozoites and blood derived antigenic material having the properties described herein for the 5.1-1 epitope-containing naturally occurring antigenic material. Thus the reactivity with sporozoite may be tested by the ability to stain either air dried or glutaraldehyde fixed sporozoites by indirect immunofluorescence, for example as described in Example 3. The reactivity with the appropriate type of blood derived antigenic material having a molecular weight in the range 20,000 to 25,000 daltons (23,000 daltons for the 5.1-1 epitope) may be tested, for example as described in Example 4.

Once one hybridoma secreting monoclonal antibodies specific for the epitope 5.1-1 has been prepared it may, if desired, be utilised in preparing alternative hybridomas of similar epitopic specificity. Monoclonal antibody specific for the 5.1-1 epitope produced by such alternative hybridomas may be of value in the isolation of antigenic materials or more particularly in other contexts, for example for the adjuvant properties. Such alternative hybridomas may, for instance, be obtained by fusing spleen cells from Balb/c mice immunised with antigenic materials containing the 5.1-1 epitope purified using antibody produced by an existing hybridoma, for example the 5.1-1 hybridoma, one suitable procedure employing the 5.1-1-Sepharose immunoadsorbent of Example 5. Hybridomas secreting antibodies with the same antigen-combining site as the antibodies secreted by the hybridoma 5.1-1 but with different heavy chain isotypes may be generated by screening for, and the subsequent isolation of, somatic variant cells from the hybridoma 5.1-1 using, for example, a fluorescence-activated cell sorter such as is described by Dangh et al., Cytometry, 1982, 2, 395 or Oi et al., Nature, 1984, 307, 136. The similarity of epitopic specificity of the antibodies produced by such alternative hybridomas may be confirmed by the procedures already described above. Variants of the hybridoma 5.1-1 of of particular interest are the isotype (heavy chain) variants.

It will be appreciated that hybridoma cell lines producing an antibody specific for different epitopes than the 5.1-1 epitope which are present in intra-erythrocytic stage antigens in alternative strains of P. falciparum are of value in similar contexts to the hybridomas producing antibody specific for the epitope 5.1-1. In particular, such hybridomas are of value in the production of alternative antigenic materials containing such epitopes other than the 5.1-1 epitope which are shared with the sporozoite surface. Following our finding that such shared epitopes exist, hybridomas producing antibodies specific for intra-erythrocytic stage epitopes may now be prepared by techniques analogous to those described herein for the preparation of the hybridoma secreting antibodies specific for the epitope 5.1-1, but utilising as the immunising agent alternative strains of P. falciparum containing a different epitope than the 5.1-1 epitope. In particular, suitable immunocyte cells for fusion may be produced by immunisation with SDS-purified parasite proteins in the size-range of 20,000 to 25,000 daltons as described above, and the desired hybridomas selected by the reactivity of the monoclonal antibodies which they produce both with such antigens and with sporozoites.

The present invention thus includes a hybridoma cell line which produces an antibody which is reactive with both the antigenic material as defined hereinbefore and the sporozoite surface of the parasite, and also any such antibody itself. In particular, the invention includes a hybridoma cell line which produces an antibody which is specific for the epitope 5.1-1 as described herein, for example the hybridoma cell line 5.1-1, and also any such antibody itself. Such hybridomas are preferably in substantially pure fom, i.e. being substantially free from any other hybridoma producing antibodies of different specificity, and the antibodies themselves are again preferably in substantially pure form, i.e. being substantially free from antibodies of a different specificity.

The hybridoma cell line 5.1-1, which exemplifies hybridomas producing antibody specific for the epitope 5.1-1, was deposited with the National Collection of Animal Cell Cultures (now the European Collection of Animal Cell Cultures) at the PHLS Centre for Applied Microbiology and Research, Porton Down, Salisbury, England, on 1st October 1984 under the accession number 84091801.

As regards the general properties of the cell line 5.1-1, particularly as compared with the NS-1 myeloma from which it derives, the following may be stated. The cell line 5.1-1 grows in HAT medium (i.e. in a supplemented culture medium, for example as described in Example 1, to which are added 13.61 mg/litre hypoxanthine, 0.176 mg/litre aminopterin and 3.88 mg/litre thymidine) and secretes mouse IgG1. Its appearance and growth characteristics in non-selective media (i.e. not a HAT or similar medium) are similar to those of the NS-1 myeloma cell line.

The following discussion relating to the production of antibodies from hybridomas according to the present invention and their use, particularly in the production of antigenic materials, is for simplicity presented in terms of antibodies specific for the epitope 5.1-1 and the production of antigenic materials containing that epitope. It will be appreciated, however, that in general terms the discussion is equally applicable to antibodies specific for other such intra-erythrocytic stage epitopes as 5.1-1 which share an epitope with sporozoites and to the production of antigenic materials containing such other epitopes.

Hybridomas secreting antibodies specific for an intra-erythrocytic stage epitope as described herein-

EP 0 153 188 B1

before, for example the hybridoma cell line 5.1-1, may be maintained by growth in various forms of nutrient culture medium. Accordingly, the present invention extends to a cell culture system comprising cells of a hybridoma secreting antibodies specific for an intra-erythrocytic stage epitope as described hereinbefore, for example the hybridoma cell line 5.1-1, in a nutrient culture medium therefor. Such a maintenance cell-culture system is conveniently an in vitro one, the culture medium being an essentially synthetic medium although it may of course contain ingredients obtained from a natural source such as serum. An example of such a culture medium is RPMI 1640 with a serum supplement such as 10 to 20% v/v of foetal calf serum, for example at 10-15% v/v. Alternatively, it may be possible to condition the cells to a lower serum level than this or to the use of the Iscove modification containing no serum (Iscove and Melchers, Journal of Experimental Medicine, 1978, 147, 923). A supplement of antibiotic is preferably used in the culture medium, for example of penicillin and streptomycin. Other supplements which are preferably also included are sodium pyruvate and glutamine. It will be appreciated that, instead of RPMI 1640, the use of various other media commonly used in cell culture and described in the literature of this art may be considered, for example Dulbecco's modification of Eagle's minimum essential medium with a serum supplement, etc. Static cell cultures may conveniently be maintained at a density of 2 to 6 x $10^5$ cells/ml by dilution into fresh medium every 24 to 72 hours, harvesting being effected once sufficient cells are generated. The cell line 5.1-1, for example, has proved to be stable to growth in static culture for at least two months but it is desirable for the hybridoma cells to be recloned periodically if growth in tissue culture is to be maintained for a prolonged period. The 5.1-1 hybridoma cells show a good level of stability to storage in liquid nitrogen.

The production of antibodies by a hybridoma secreting antibodies specific for an intra-erythrocytic stage epitope as described hereinbefore, for example the hybridoma cell line 5.1-1, may be effected through its culture in either an in vitro or an in vivo culture medium therefor. The present invention thus includes a process for the production of antibodies which comprises culturing cells of a hybridoma secreting antibodies specific for an intra-erythrocytic stage epitope as described hereinbefore, for example the hybridoma cell line 5.1-1, in an in vitro or in vivo culture medium therefor and thereafter isolating the antibodies from said medium.

The choice between an in vitro and an in vivo procedure depends on various factors. Thus in vitro culture may be indicated where the use for which the antibodies are required is of an immunological rather than a chemical nature and the presence of mouse or other immunoglobulin contaminants from in vivo growth in an animal is undesirable. In vivo culture does, however, have the particular advantage that significantly higher levels of antibody per ml are generally obtained (in serum and/or ascitic fluid) as compared with an in vitro culture medium. In vivo culture is preferably carried out in a mouse, particularly a Balb/c mouse, although culture in other strains of mice or other animals, for example rats, may be considered, particularly by the use of irradiation and/or of immunosuppressive drugs.

In the case of an in vitro system for antibody production, the culture medium used may be similar to that described above in relation to a cell maintenance culture system but the serum levels are preferably as low as possible, for example being from 5-10% v/v. Alternatively the use of the Iscove serum-less modification may again be considered.

Examples of suitable tissue culture procedures of antibody production are massive growth in spinner containers and other known mass culture procedures which are well documented in the art. The in vivo procedure conveniently comprises the intra-peritoneal inoculation of inbred, Balb/c, mice to produce a solid or ascitic tumour. Prior to inoculation the animal may conveniently be immunosuppressed and/or treated with a drug such as pristane to induce ascitic fluid secretion. After a suitable period of growth of the tumour the animal is killed and the ascites and/or serum are collected for isolation of the antibody, optionally following a period of collection of ascitic fluid from the living animal in the case of an ascitic tumour. An ascitic tumour thus has the potential of producing a higher amount of fluid but the value of this technique will depend upon the concentration of antibody present in the ascites and/or serum. Isolation of the antibody from either an in vitro or an in vivo procedure is conveniently effected by procedures described in the art including precipitation, dialysis, chromatography including the use of immunoadsorbents, and the use of membrane filters.

Monoclonal antibodies produced by hybridomas secreting antibodies specific for an intra-erythrocytic stage epitope as described hereinbefore, for example the hybridoma cell line 5.1-1, show the unusual characteristic among monoclonal antibodies raised against the intra-erythrocytic stages of the malaria parasite of giving a strongly positive reaction to sporozoite preparations. Thus, for the hybridoma cell line 5.1-1, activity has been observed with such preparations at a 1 in $10^6$ dilution level of ascitic fluid containing the antibodies whilst a variety of other monoclonal antibodies raised against intra-erythrocytic stages have been tested and found not to react with sporozoites at a dilution level of 1 in $10^2$. Antibodies specific for the epitope 5.1-1 have been found to show the same high level of activity against intra-erythrocytic stages for

6

most, but not all, of a wide range of P. falciparum isolates from different regions. Both air dried and glutaraldehyde fixed sporozoites are stained by indirect immunofluorescence with such monoclonal antibodies. Since the latter method of preparation only allows antibody to bind to surface determinants, the clear microscopic image it provides with the 5.1-1 epitope-specific antibodies is strong evidence that the epitope to which the antibody is binding is located at the sporozoite surface. By contrast, the indirect immunofluorescence of the intra-erythrocytic parasites suggests that the epitope is associated with the parasite and/or parasitophorous vacuole membrane(s) and with parasite derived inclusions in the infected erythrocyte, where such occur, but usually not with the surface of the infected erythrocyte.

Monoclonal antibodies obtained from hybridomas secreting antibodies specific for an intra-erythrocytic stage epitope as described hereinbefore, for example the hybridoma cell line 5.1-1, are of potential interest as an immunodiagnostic reagent. The immunofluorescence pattern of the 5.1-1 epitope-specific antibodies reacted with intra-erythrocytic stages strongly suggests that the naturally occurring 5.1-1 epitope-containing antigen (known from extensive biochemical studies to be synthesised in the parasite) is transported to the membrane of the parasitiphorous vacuole and thence through the cytosol of the host erythrocyte. It is therefore entirely possible that the natural antigen containing the 5.1-1 epitope is secreted by parasitised erythrocytes and, in addition, this antigen may be released by incoming sporozoites. It is consistent with this view that many antibodies are found in human immune sera directed against the 5.1-1 epitope-containing antigen. Detection of circulating antigen with 5.1-1 epitope-specific antibodies could therefore provide a route for early diagnosis, for example by ELISA. For use as diagnostic reagents the antibodies are normally formulated in a suitable diluent or carrier, for example phosphate-buffered physiological saline. The present invention thus also includes a composition comprising monoclonal antibodies specific for an intra-erythrocytic stage epitope as described hereinbefore, for example antibodies secreted by the hybridoma cell line 5.1-1, in a physiologically acceptable diluent or carrier. Other potential uses include the use of antibodies in the form of such a composition for passive immunisation (although active immunisation with an antigenic material is preferred) and also the use of antibodies of certain isotypes, again conveniently in the form of such a composition, to exert an adjuvant effect, as discussed hereinafter.

The major use of the monoclonal antibodies obtained from hybridomas secreting antibodies specific for an intra-erythrocytic stage epitope as described hereinbefore, for example the hybridoma cell line 5.1-1, does, however, lie in their value in the production of antigenic materials containing the epitope for which the antibody is specific. The monoclonal antibody may be used in two main ways for the production of antigenic materials containing the epitope in question. Firstly the antibody may be used in the production of such antigenic materials deriving from nature. The antibody may be used either solely in the detection of the desired material or more actively in its isolation and/or purification.

It has been found possible using 5.1-1 epitope-specific antibodies and a P. falciparum isolate designated K1 from Thailand to identify a naturally occurring antigenic material having a molecular weight estimated by SDS-PAGE as 23,000 daltons (22,000 to 24,000 daltons within the range of accuracy of the method), the procedure used for this estimation of molecular weight being that described by Laemmli, Nature, 1970, 227, 680. Accordingly, the antigenic materials as defined hereinbefore particularly comprise that antigenic substance derivable from P. falciparum having a molecular weight of 23,000 daltons and containing an epitope which is shared with an antigenic material located on the sporozoite stage of the parasite.

This antigenic substance may conveniently be obtained by the cleavage of P. falciparum infected erythrocytes, conveniently using an erythrocyte preparation comprising a substantial proportion of the pigment-containing mature intra-erythrocytic stages of the parasite which contain higher levels of the antigen, the substance being most abundant in trophozoites and schizonts. Following cleavage, the proteins are extracted from the released parasites and separated, the desired substance conveniently being detected among the separated proteins by use of a 5.1-1 epitope-specific antibody. The cleavage of the erythrocytes may conveniently be achieved with a saponin, for example at 4°C using a concentration of about 0.1%, followed by extraction with a surface active agent, for example an ionic agent such as SDS. The separation of the proteins may then conveniently be achieved by SDS-PAGE followed by Western blotting onto nitrocellulose, the 23,000 dalton molecular weight antigenic substance occuring as a single band identifiable by the ELISA technique utilising 5.1-1 epitope-specific antibody.

Since the 23,000 daltons molecular weight antigenic substance cannot readily be isolated from the nitrocellulose, it is preferred to utilise an affinity chromatography procedure involving 5.1-1 epitope-specific antibody to effect isolation of an antigenic material containing the 5.1-1 epitope. It has been found, however, that such a procedure leads to an antigenic substance which, instead of having a molecular weight of 23,000 daltons has a molecular weight estimated by SDS-PAGE as 22,000 daltons (21,000 to 23,000 daltons within the range of accuracy of the method), apparently due to some specific cleavage during the purification procedure. Accordingly, the antigenic materials as defined hereinbefore also particularly comprise that antigenic substance derivable from P. falciparum having a molecular weight of 22,000 daltons and containing an epitope which is shared with an antigenic material located on the sporozoite stage of the parasite. It is to be anticipated that other naturally occuring antigenic materials with a molecular weight in the range of 20,000 to 25,000 daltons containing a different epitope to the epitope 5.1-1 will also yield similar derivative products of slightly lower molecular weight.

For use in an affinity chromatography purification procedure the 5.1-1 epitope-specific antibody may conveniently be attached to a support material in order to provide an immobilised solid phase with which the antigenic substance is contacted. The present invention thus includes monoclonal antibodies specific for a shared epitope as described hereinbefore, for example antibodies specific for the epitope 5.1-1 such as those secreted by the hybridoma 5.1-1, in insolubilised form through immobilisation on a solid support material.

The solid phase may comprise one of a variety of forms of polymeric material suitable as the basis of an immunoadsorbent. Such materials are described in the literature in relation to gel filtration chromatography of proteins and include carbohydrate materials based upon dextran, agar or agarous and also polyacylamide based materials, cross-linking conveniently being used to impart the required degree of exclusion of molecules depending upon size and shape. Examples of such materials are marketed under the trade names Sepharose (agarose based), Sephadex (dextran based) and Sephacryl (allyldextran based with cross-linking by an acrylamide derivative). The agarose material marketed under the trade name Sepharose 4B is one example of a suitable material. The antibodies may be attached to the support material through the use of one of various methods described in the literature by which proteins or other materials may be attached to insoluble solid phase supports of use in affinity chromatography. The chemical reactions described effect covalent bond attachment, but by a non-denaturing methodology so that destruction of the attached labile substances is minimised. Examples of suitable linking agents include the 1,4-bis-(2,3-epoxypropoxy)-butane type of linking agent and particularly cyanogen bromide, these linking agents being suitable for use with non-derivatised support materials such as those carbohydrate materials specified above. Alternatively, various derivatised support materials containing a reactive functional group may be used with or without a separate linking agent, depending on their particular nature. Thus, for example, the material CNBr-activated Sepharose 4B marketed by Pharmacia is particularly useful.

For the affinity chromatography purification procedure, infected erythrocytes again conveniently provide the source material, and in this case the erythrocyte cleavage and parasite protein extraction are more conveniently achieved simply by contact with a suitable surface active agent-containing medium which avoids any extensive degree of proteolysis and denaturation. Non-ionic detergents are preferred as the surface active agent, such as those of the derivatised ethylene oxide condensate type, with Nonidet P40 (an octyl phenol ethylene oxide condensate) being of particular interest, at a suitable concentration usually between 0.01 and 5% v/v, for example 1% v/v. A suitable medium consists of a buffer of 50mM 2-amino-2-hydroxymethylpropane 1,3-diol hydrochloride (Tris-HCl) of pH 8.0, 5mM ethylenediamine N,N'-tetraacetate (EDTA), 5mM ethyleneglycol bis-(aminoethyl ether) N,N'-tetraacetate (EDTA), 5mM iodoacetamide, 1mM phenylmethyl sulphonyl fluoride and 0.1mM tosyl-L-lysine chloromethyl ketone (TLCK) containing 1% v/v NP40. The protein extract is applied to the affinity chromatography column which is then processed to provide the desired protein in purified form. Such processing conveniently involves washing to remove proteins which are not specifically bound, for example using the same buffer as that used previously but lacking the surface active agent, followed by elution of the specifically bound protein through the use of an agent which will disrupt the complex on the column. Suitable conditions which preserve the immunogenicity of the protein include the use of urea, for example at 8M concentration. Suitable methods for recovery of the protein from the eluate include concentration and dialysis.

The present invention thus further includes a process for the preparation of an antigenic material as defined hereinbefore which comprises absorbing said antigenic material or a precursor thereof on a solid support material which carries monoclonal antibodies reactive with both the sporozoite surface of a parasite of the species Plasmodium falciparum and with an antigenic material of a molecular weight as determined by SDS-PAGE in the range of 20,000 to 25,000 daltons comprising a sequence of amino acid residues which occurs in intra-erythrocytic forms of the parasite, and thereafter eluting the material from the support material.

The antigenic substances of molecular weight 23,000 and 22,000 daltons are now further described, any one or any combination of the properties described for these substances being available for their characterisation in addition to the properties mentioned previously.

Both substances are proteinaceous, i.e. substantially, but not necessarily exclusively, of protein form. The 5.1-1 epitope, which is not necessarily the sole epitope contained by these subtances, is present in their primary amino acid sequence and does not require post translational processing for its generation. This is shown by the fact that the primary translation product made in vitro by rabbit reticulocyte lysates can be immunoadsorbed by 5.1-1 epitope-specific antibodies as described hereinafter. The substances are encoded by the parasite and contain methionine as evidenced by (a) metabolic labelling in live parasites with $^{35}$S-methionine and (b) labelling with $^{35}$S-methionine by in vitro translation of total P. falciparum mRNA as discussed hereinafter. The antigenic substances of molecular weight 23,000 daltons is synthesised throughout the erythrocytic cycle of P. falciparum as about 0.1% of the total protein synthesised, but disappears from the parasite at the end of the cycle (after rupture of the mature schizont), presumably being degraded. The substance accumulates throughout the cell cycle, being present at levels detectable by indirect immunofluorescence and immunodetection of Western blots using 5.1-1 epitope-specific antibody only for late stages of the erythrocytic cycle (mature trophozoites and schizonts). The substances are strongly recognised by human sera from individuals living in endemic areas, as evidenced by their detection with such sera after SDS-PAGE and Western blotting.

It will be appreciated that various other antigenic materials of lower molecular weight containing the 5.1-1 epitope may also be prepared by treatment of the naturally occurring material of 23,000 daltons molecular weight. Moreover, as discussed hereinafter, it is sometimes desirable to attach antigenic materials containing the 5.1-1 or other such epitope to a carrier such as a protein in order to enhance their immunogenicity and this may even produce materials of higher molecular weight than the naturally occurring materials. Thus, whilst the molecular weight range of 20,000 to 25,000 daltons given hereinbefore is a preferred one, it will be appreciated that antigenic materials according to the present invention can be of a wide range of size.

The second way in which 5.1-1 epitope-specific antibodies may be used in the production of antigenic materials containing the 5.1-1 epitope involves the use of genetic engineering procedures for the synthesis of protein or peptide substances containing the epitope. In one particular aspect, therefore, the present invention comprises an antigenic material suitable for use in vaccination against malaria as defined hereinbefore being a synthetic immunogenic peptide or protein, especially one produced by genetic engineering.

One approach using genetic engineering procedures involves the use of P. falciparum mRNA which and its translation in rabbit reticulocyte extracts by the method of Pelham and Jackson, European Journal of Biochemistry, 1976, 67, 247. The products obtained by this procedure may be processed analogously to the products produced in vivo, particularly by the use of 5.1-1 antibody affinity chromatography. In this case the affinity chromatography procedure yields a substance of molecular weight 24,500 daltons (23,500 to 25,500 daltons within the range of accuracy of the method). This substance must presumably be processed in vivo to the 23,000 dalton molecular weight material. As the translation of the mRNA is known to produce proteins which are not processed or modified, the specific binding of the 24,500 daltons molecular weight protein by the 5.1-1 affinity chromatography column indicates that the epitope is determined by the primary amino acid sequence of the antigenic materials containing the epitope.

A second approach using genetic engineering procedures involves the cloning of cDNA molecules corresponding to residues of the sequence amino acids present in the naturally occurring antigenic materials described hereinbefore in order to be able to produce synthetic antigenic materials falling within the scope of the present invention. Such an approach has been employed with an antigen of P. knowlesi by Ellis et al., Nature, 1983, 302, 536 and may also be applied in the context of the present invention following identification of the existence of the intra-erythrocytic stage antigens containing an epitope shared with the sporozoite stage of the parasite.

By the application of genetic engineering techniques and starting with a P. falciparum isolate designated K1 from Thailand we have identified a DNA sequence which is believed to correspond to most if not all of that of the structural gene for the naturally occurring P. falciparium intra-erythrocytic stage antigen which contains the 5.1-1 epitope, in its primary translation product form. The amino acid sequence corresponding to this polynucleotide sequence thus defines this antigen. It should be pointed out that although the natural primary translation product has a molecular weight, as estimated by SDS-PAGE, of about 24,500 daltons, the molecular weight achieved by summation of the individual amino acid residues of this sequence is no greater than 17,000 daltons. Now, it is possible that the naturally occurring material, although being proteinaceous may be glycosylated, whilst the genetically engineered material is not. However, that this is not the primary cause for the discrepancy in molecular weight is shown by the fact that the genetically engineering material, itself, shows a molecular weight of about 24,000 daltons as determined by SDS-PAGE (as indicated before, for figures of this magnitude the accuracy of the SDS-PAGE method is generally recognised as being within ± 1,000 daltons and the difference of 500 daltons observed between the molecular weights of the natural primary translation product and the genetically engineered antigen may not represent any true difference in size). The explanation for the discrepancy between the molecular weights obtained by measurement and by summation instead apparently lies in the consistent, significant over-estimates of molecular weight which have previously been reported in the literature for Plasmodium antigens when using SDS-PAGE.

Accordingly the anitgenic material suitable for use in vaccination against malaria as defined hereinbefore may comprise the whole or a portion or portions of the following sequence of one hundred and sixty-two amino acid residues:

Met-Lys-Ile-Leu-Ser-Val-Phe-Phe-Leu-Ala-Leu-Phe-Phe-Ile-Ile-Phe-

Asn-Lys-Glu-Ser-Leu-Ala-Glu-Lys-Thr-Asn-Lys-Glu-Thr-Gly-Ser-Gly-

Val-Ser-Ser-Lys-Lys-Lys-Asn-Lys-Lys-Gly-Ser-Gly-Glu-Pro-Leu-Ile-

Asp-Val-His-Asp-Leu-Ile-Ser-Asp-Met-Ile-Lys-Lys-Glu-Glu-Glu-Leu-

Val-Glu-Val-Asn-Lys-Arg-Lys-Ser-Lys-Tyr-Lys-Leu-Ala-Thr-Ser-Val-

Leu-Ala-Gly-Leu-Leu-Gly-Val-Val-Ser-Thr-Val-Leu-Leu-Gly-Gly-Val-

Gly-Leu-Val-Leu-Tyr-Asn-Thr-Glu-Lys-Gly-Arg-His-Pro-Phe-Lys-Ile-

Gly-Ser-Ser-Asp-Pro-Ala-Asp-Asn-Ala-Asn-Pro-Asp-Ala-Asp-Ser-Glu-Ser-Asn-Gly-Glu-Pro-Asn-Ala-Asp-Pro-Gln-Val-Thr-Ala-Gln-Asp-Val-

Thr-Pro-Glu-Gln-Pro-Gln-Gly-Asp-Asp-Asn-Asn-Leu-Val-Ser-Gly-Pro-

Glu-His.

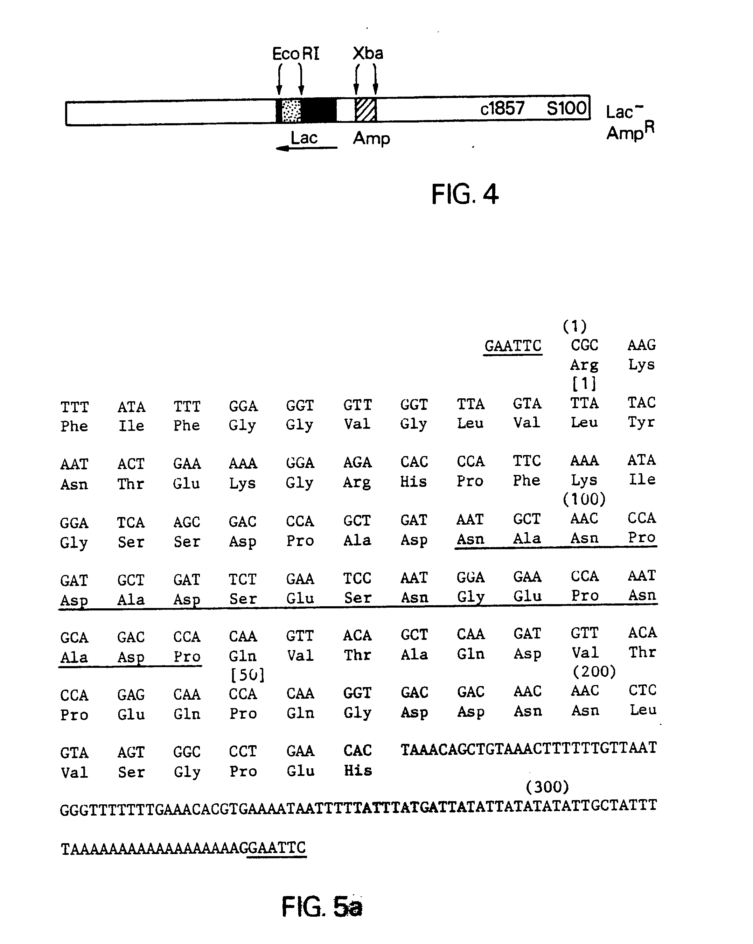

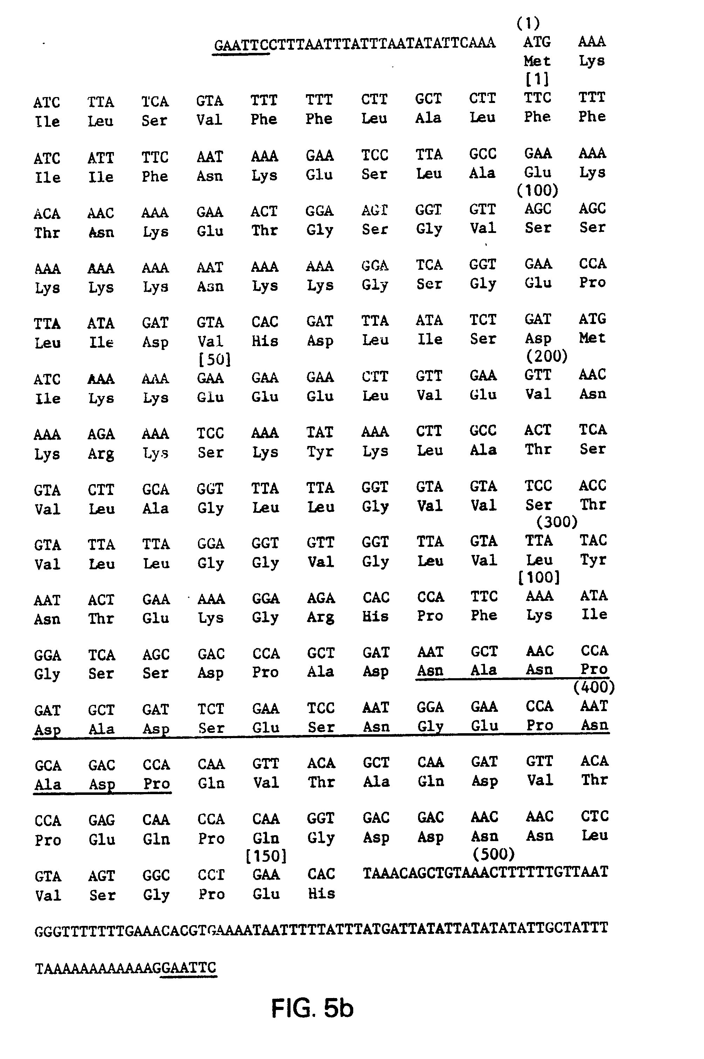

It will be appreciated that the whole of the amino acid sequence given above need not be present for the antigenic material to be suitable for use in vaccination against malaria. An antigenic material comprising a smaller amino acid sequence may be of value immunogenically due to the presence in that sequence of one or more intra-erythrocytic epitopes, particularly the 5.1-1 epitope. Such an epitope is more likely to correspond to a sequence or a combination of sequences consisting in total of at least five and especially at least ten of the amino acid residues indicated, particularly fifteen or more residues. (Where more than one non-consecutive sequence of the amino acid residues indicated is present, each of these sequences is also likely to contain at least four or five residues.) Thus, without thereby limiting the scope of the present invention, it may be stated that the 5.1-1 epitope is believed to be constituted by the sequence of eighteen amino acid residues (underlined in Figure 5) Asn-Ala-Asn-Pro-Asp-Ala-Asp-(Ser-Glu-Ser)-Asn-Gly-Glu-Pro-Asn-Ala-Asp-Pro, this sequence having homology with the tandemly repeated tetramer which is present in the circumsporozoite protein. This tetramer consists of the amino acid residues Asn-Ala-Asn-Pro and the sequence of amino acid residues consisting of this repeated tetramer, and a variation thereon in which the Ala is replaced by Val and the second Asn by Asp to give the sequence Asn-Val-Asp-Pro, is believed to constitue the shared epitope in its sporozoite form. It will be seen that although there is a degree of similarity, the intra-erythrocytic and sporozoite epitopes are not identical.

Among the eighteen amino acid residues of the intra-erythrocytic stage epitope, it is believed that the fifteen residues not included in the brackets are of particular importance and that the three residues Ser-Glu-Ser may simply provide an appropriate juxtaposition between the sequence of seven and eight residues on either side thereof. It is possible therefore that these three residues could be replaced by others, particularly by other residues generally regarded in the art as being related thereto, so that the replacement of Ser by Ala and of Glu by Asp may for example be considered. Alternatively it may even be possible to remove this sequence of three residues entirely, although it is believed that the presence of a group of amino acid residues, particularly of three residues, separating the Asn-Ala-Asn-Pro-Asp-Ala-Asp and Asn-Gly-Pro-Asn-Ala-Asp-Pro sequences is probably desirable.

It should be stressed, however, that the full amino acid sequence is believed to contain not only the 5.1-1 epitope and that the presence of other epitopes in the sequence is believed to be of value in providing an antigenic material of maximum efficacy in vaccination both against P. falciparum sporozoites, which share the 5.1-1 epitope, and intra-erythrocytic forms of this parasite. Thus an antigenic material containing a large part of the amino acid sequence specified when administered in vivo would be expected to induce the formation of a plurality of antibodies, some of which would be specific for the 5.1-1 epitope and would react with both sporozoites and intra-erythrocytic forms of the parasite, and others of which would also be expected to react with intra-erythrocytic forms of the parasite, thereby enhancing the protection afforded by the use of a vaccine containing the antigenic material.

Although it is therefore possible to utilise antigenic materials containing only a part of the amino acid sequence given hereinbefore, it is likely that any significant reduction, even with retention of the 5.1-1 epitope, may lead to loss of the sites of other valuable epitopes present in the full sequence. In general, therefore, although the synthetic antigenic materials according to the present invention may contain a somewhat reduced amino acid sequence, for example one lacking certain of the residues located at the N- and/or the C- terminus of the sequence, such antigenic materials of particular interest have a molecular weight, as estimated by SDS-PAGE, which is not less than 20,000 or 22,000 daltons, for example lying in the range of 22,000 to 24,000 daltons specified hereinbefore.

Examples of smaller antigenic materials than that containing the full amino acid sequence given hereinbefore include the proteins of molecular weights 22,000 and 23,000 daltons, as estimated by SDS-PAGE, which have been obtained in addition to a protein of molecular weight 24,000 daltons on expression of the gene present in the clone λAg5.1(9), as discussed hereinafter. A further example of an antigenic material containing a part of the full amino acid sequence is one containing the whole or a part of the amino acid sequence corresponding to that partial sequence of the gene which is present in the alternative clone λAg5.1(8), as described hereinafter. This smaller amino acid sequence corresponds to the sixty-nine C-terminal amino acids of the full sequence, having an N-terminal glycine residue which corresponds to the ninety-fourth amino acid residue of the full sequence, and a C-terminal histidine residue. Another smaller antigenic material containing a part of the full sequence is a peptide corresponding to the fifteen amino acid residues identified hereinbefore as being responsible for the specificity of the 5.1-1 epitope, preferably together with a group of separating amino acid residues, for example three residues and particularly Ser-Glu-Ser as present in nature.

Although antigens having a molecular weight in a range from 20,000 to 25,000 daltons should be fully immunogenic, if smaller antigenic materials are used, such as a peptide corresponding to the 5.1-1 epitope, these may have molecular weights which are too small for satisfactory functioning as an immunogen. This is of course the case not only for synthetic antigenic materials but also for materials produced by cleavage of the naturally ocurring antigens. It will be appreciated, therefore, that antigenic materials according to the present invention include those in which a peptide or protein corresponding to the whole or particularly a part of the full amino acid sequence is attached to a suitable carrier, particularly a carrier protein such as the natural proteins bovine serum albumin and more particularly ovalbumin and keyhole limpet hemocyanin, and synthetic polypeptides such as polylysine.

The initial requirement for the application of the second approach using genetic engineering procedures referred to hereinbefore is the isolation and identification of the whole or a part of the gene coding for the naturally occurring antigenic material which it is required to produce in whole or in part. It will be appreciated that a number of approaches are possible to this problem, as will be apparent to the man skilled in the art. Thus, it is possible to make use of Plasmodium genomic DNA, this being cut into appropriate fragments and inserted directly into an expression vector, for example a phage vector such as λgt11 or a derivative thereof. However, the following procedure employing RNA to provide cDNA which is inserted into a phage vector has enabled the isolation of a gene product corresponding to most, if not all, of the structural gene coding for the naturally occurring intra-erythrocytic stage P. falciparum antigen carrying an epitope which is shared with the surface of sporozoites, such a procedure also being applicable to the isolation of the gene coding for the similar antigens of other species of Plasmodium.

Total messenger RNA from erythrocytic stages of the parasite is converted to cDNA and linkers containing restriction sites for the endonuclease EcoRI are attached to the cDNA termini. Following treatment with EcoRI, the resultant molecules are inserted into the sole EcoRI site of a selected phage λ vector and packaged into λ capsids in vitro. A particularly convenient vector to use is a derivative of λgt11 known as λgt11-Amp3 which is described by Kemp et al, Proceedings of the National Academy of Sciences of the USA, 1983, 80, 1194 and which carries a gene conferring ampicillin-resistance between the lacZ and the att site of the phage. The EcoRI site into which the cDNA molecules are inserted is located within the gene encoding β-galactosidase (lacZ) so that the cDNA can be transcribed and translated.

The phage carrying the cDNA fragments is used to infect an E.coli host, a convenient host being the E.coli Y1090 described by Young and Davis, Science, 1983, 222, 778. The phage plaques produced in this way are then screened to identify clones which express peptides/proteins of interest. A convenient approach to such screening involves the use of a monoclonal antibody specific for the epitope contained in these antigenic materials, for example in the case of the 5.1-1 epitope of P. falciparum the monoclonal antibody produced by the hybridoma 5.1-1 or any other monoclonal antibody of similar specificity produced by another hybridoma may be used as a probe for screening the clones. As an alternative to the direct use of

EP 0 153 188 B1

the monoclonal antibody specific for the epitope as a probe, such an antibody may be used to purify the antigen in question in order to raise a polyclonal antibody preparation specific for a naturally occurring antigen containing the epitope.

The procedure described above is illustrated in more detail in the Examples and has led to the isolation of two phage clones of particular interest. These clones have been designated by us as λAg5.1(8) and λAg5.1(9), the latter having been deposited as an E.coli Y1090 lysogen with the National Collections of Industrial and Marine Bacteria in Aberdeen, Scotland under the more detailed designation ΔlacU169, Δlon, araD139, strA, thi, supF, trpC22::Tn10/λIH9 on 14th September 1984, with the accession number NCIB 12016. The lysogen consists of the E.coli Y1090 [Δlac U169, Δlon, araD139, strA, thi, supF [trp C22::Tn10] (pMC9 (lacI⁺, amp^R))] strain of Young and Davis, ibid, lysogenised by the phage λgt11-Amp3 (lac5, nin5, cl857, S100, amp^R) of Kemp, ibid, containing an insert of P. falciparum DNA, as described hereinafter, at the single EcoRI site thereof. The lysogen cells show the usual E.coli morphology, but with the temperature sensitivity usually conferred by lysogenisation, coupled with the properties conferred by the extra DNA which is present.

The inserts in the two clones λAg5.1(8) and λAg5.1(9) have been investigated by the usual procedure of growing the phage in the form of E.coli lysogens, inducing lysis, purifying the phage and extracting DNA, cleaving the DNA to isolate the fragments between the EcoRI sites and then sequencing these fragments. The insert in clone λAg5.1(8) is illustrated in Figure 4 whilst that in clone λAg5.1(9) is illustrated in Figure 5. In each case the GAATTC nucleotide sequence which constitutes an EcoRI site is shown underlined at both the N- and the C-terminus, although it will be appreciated that a part of each of these sequences is derived from the phage λgt11-Amp3 so that the insert itself contains in total only one set of GAATTC nucleotides.

Both the clones have been shown to express a protein which will react with antibodies produced by the 5.1-1 hybridoma and therefore contain the 5.1-1 epitope, the insert in clone λAg5.1(8) overlapping for a very large part thereof with part of the nucleotide sequence of the clone λAg5.1(9). It is believed that most, if not all, of the structural gene for the naturally occurring intra-erythrocytic stage P. falciparum antigen containing an epitope occurring in intra-erythrocytic stages of the parasite and shared with the surface of sporozoites corresponds to the nucleotide sequence in the λAg5.1(9) clone insert starting with the ATG codon adjacent to the 5' terminus thereof and finishing with the CAC codon adjacent to the 3' terminus thereof.

The present invention thus further includes recombinant DNA comprising a cloning vehicle sequence and DNA which comprises either the whole or a portion or portions of the following nucleotide sequence including a sequence coding for an epitope present in intra-erythrocytic forms of a parasite of the species Plasmodium falciparum which is shared with an antigenic material located on the sporozoite stage of the parasite:

| ATG | AAA | ATC | TTA | TCA | GTA | TTT | TTT | CTT | GCT | CTT |
|-----|-----|-----|-----|-----|-----|-----|-----|-----|-----|-----|
| TTC | TTT | ATC | ATT | TTC | AAT | AAA | GAA | TCC | TTA | GCC |
| GAA | AAA | ACA | AAC | AAA | GAA | ACT | GGA | AGT | GGT | GTT |
| AGC | AGC | AAA | AAA | AAA | AAT | AAA | AAA | GGA | TCA | GGT |
| GAA | CCA | TTA | ATA | GAT | GTA | CAC | GAT | TTA | ATA | TCT |
| GAT | ATG | ATC | AAA | AAA | GAA | GAA | GAA | CTT | GTT | GAA |
| GTT | AAC | AAA | AGA | AAA | TCC | AAA | TAT | AAA | CTT | GCC |
| ACT | TCA | GTA | CTT | GCA | GGT | TTA | TTA | GGT | GTA | GTA |
| TCC | ACC | GTA | TTA | TTA | GGA | GGT | GTT | GGT | TTA | GTA |
| TTA | TAC | AAT | ACT | GAA | AAA | GGA | AGA | CAC | CCA | TTC |
| AAA | ATA | GGA | TCA | AGC | GAC | CCA | GCT | GAT | AAT | GCT |
| AAC | CCA | GAT | GCT | GAT | TCT | GAA | TCC | AAT | GGA | GAA |
| CCA | AAT | GCA | GAC | CCA | CAA | GTT | ACA | GCT | CAA | GAT |
| GTT | ACA | CCA | GAG | CAA | CCA | CAA | GGT | GAC | GAC | AAC |
| AAC | CTC | GTA | AGT | GGC | CCT | GAA | CAC |     |     |     |

12

or which comprises an equivalent nucleotide sequence coding for the same amino acid residues.

It will be appreciated that the usage employed in this specification is the common one of showing only a single strand of the DNA sequence although the DNA will of course exist as two strands, the structure of the second strand however being dictated by that of the first so that ony one strand need be shown. Furthermore, since a particular amino acid can be encoded by more than one triplet, the invention does extend to alternative DNA which is equivalent in its coding to the whole or a part of the sequence specified. Indeed, it is possible that other strains of P. falciparum than that contained in the K1 isolate used to provide the cDNA which we have utilised might contain such DNA differing slightly in its structure from that described herein although containing equivalent codon(s) coding for the same amino acid residue(s) corresponding to the 5.1-1 epitope. In practice, however, it will usually be much simpler to utilise DNA comprising the natural sequence of codons.

Smaller nucleotide sequences than the whole which are of particular interest will correspond to the antigenic materials discussed hereinbefore which contain only a portion or portions of the amino acid sequence present in the protein which is the primary translation product. Such DNA not comprising the whole sequence may, for example, correspond to antigenic materials with a molecular weight of 22,000 to 24,000 daltons or to an amino acid sequence as discussed hereinbefore which constitutes an epitope, i.e. being a sequence or combination of sequences which are more likely to contain in total at least five and especially at least ten of the condons indicated, particularly fifteen or more codons, such as the nucleotide sequence AAT GCT AAC CCA GAT GCT GAT TCT GAA TCC AAT GGA GAA CCA AAT GCA GAC CCA (underlined in Figure 5) or other sequence coding for the same amino acid residues. In this sequence the nucleotides TCT GAA TCC joining the codons GAT and AAT, in particular, may optionally be replaced by three alternative codons coding for the three amino acid residues Ser-Glu-Ser. Alternatively, it may be possible to replace these nucleotides by others coding for different amino acid residues, particularly for a total of three residues, and it may also even be possible to omit these nucleotides completely. As discussed below, the nucleotide sequence coding for the desired antigenic material will in use generally be linked to other nucleotides serving various functions related to the expression of that material.

For expression of a desired antigenic material, such a nucleotide sequence corresponding to the structural gene or a portion thereof needs to be located in a suitable cloning vehicle. The present invention thus also includes recombinant DNA which comprises a cloning vehicle sequence and the nucleotide sequence defined above or a portion thereof, for example recombinant DNA as is present in the clones λAg5.1(8) and λAg5.1(9).

It will be appreciated that a wide variety of such cloning vehicles may be employed, for example E.coli plasmids and plasmids for use in alternative bacterial hosts, including cosmids and particularly phages. Of particular interest are lambda phage, for example phage λgt11 or a modification thereof such as λgt11-Amp3. Cloning vehicles containing the structural gene, or portions thereof, may be prepared starting with a cDNA or a genomic library as discussed hereinbefore or, much more conveniently, the deposited clone λAg5.1(9) may be used as a source of the structural gene DNA which may be inserted into an alternative cloning vehicle, for example one providing enhanced expression of the protein.

In order for the recombinant DNA comprising a cloning vehicle and the structural gene, or a portion thereof, to express the desired antigenic material it will be appreciated that the recombinant DNA should contain appropriate expression control sequences, i.e. promoter and terminator DNA sequences to effect transcription, translation stop and start signals, and optionally other regulatory sites advantageously affecting expression. An example of an additional form of regulation having beneficial effects is the ability to switch off expression of the gene during the initial stages of culture of the host. Thus, the lac operator may be used in this way if a host bacterium is used containing the lacI gene encoding the lac repressor. The lac operator is present in the λgt11-Amp3 phage whilst the E.coli Y1090 strain contains the lacI gene.

The sequences controlling transcription will usually be provided by the host/cloning vehicle DNA, for example the promotion of transcription being effected by the DNA normally controlling the transcription of β-galactosidase in the case of the λgt11-Amp3 phage. The sequences providing the translation start and stop signals however, usually may derive either from the cloning vehicle or from P. falciparum. There is a distinction between the two clones λAg5.1(8) and λAg5.1(9) referred to hereinbefore in this respect since, although both clones contain a P. falciparum derived translation stop signal (TAA), the λAg5.1(9) clone, unlike the λAg5.1(8) clone, also contains a P. falciparum derived start signal (ATG), thereby providing the P. falciparum protein unfused to other peptide material.

Recombinant DNA according to the present invention thus conveniently further comprises, in addition to the DNA coding for the P. falciparum protein or peptide, at the 5' terminus thereof, a (5' → 3') nucleotide sequence CTTTAATTTATTTAATATATTCAAA or a part thereof which provides a translation start signal, and/or at the 3' terminus thereof a (5' → 3') nucleotide sequence TAAACAGCTGTAAACTTTTTTGTTAATG-

GGTTTTTTTGAAACACGTGAAAATAATTTTTATTT ATGATTATATTATATATATTGCTATTT-TAAAAAAAAAAAAAAAAAAAG or a part thereof (for example lacking all or part of the poly A sequence), which provides a translation stop signal.

For the production of a protein by expression from the cloning vehicle, this vehicle is necessarily comprised in a host. The host can with particular convenience be a bacterium, for example a strain of Pseudomonas or Bacillus or most particularly a strain of E.coli such as E.coli Y1090, although the use of yeasts and other fungi or of mammalian cells may be considered. It will be appreciated that the particular host selected depends on the particular cloning vehicle used but, having selected an appropriate combination of cloning vehicle and host, the latter may readily be transformed by the former (or transfected or lysogenized in the case of phage cloning vehicles) using techniques known in the art. The present invention accordingly includes host cells comprising a cloning vehicle as described hereinbefore containing the previously defined nucleotide sequence or a portion thereof.

To produce an antigenic material according to the present invention a host as described above is cultured under suitable conditions to effect expression from the cloning vehicle. The conditions selected will be appropriate to the host/cloning vehicle combination so that for an E.coli Y1090 phage lysogen, for example, culture may be effected in a medium such as L-broth (Difco Bacto Tryptone, 10 g; Difco Bacto Yeast Extract, 5 g; NaCl, 5 g; water to 1 litre; pH 7.2) at about 30°C in air. Where the phage vector containing the P. falciparum DNA contains the lac operator and the host contains the lacI gene then, as discussed previously, the gene may conveniently be switched off initially and expression may be induced when desired by the addition of isopropyl thio-$\beta$-D-galactosidase to the culture medium, which inactivates the lac repressor and thus switches on the gene. For isolation of the protein/peptide the medium and/or host cells are processed by appropriate means. When using a bacterial lysogen, for example, the host cells may conveniently be harvested, disrupted, for example by the use of a suitable surface active agent such as sodium dodecyl sulphate, and the products separated, for example by preparative SDS-PAGE, although alternative conventional procedures, for example those adapted to large scale production, may also be employed. Where the desired P. falciparum antigenic material is expressed as a fusion protein, as in the case with the clone $\lambda$Ag5.1(8) where a fusion product containing $\beta$-galactosidase is obtained, the fusion protein may either be cleaved or used as such (the increased size sometimes being of value for increasing immunogenicity.

As previously indicated, the use of the clones $\lambda$Ag5.1(8) and $\lambda$Ag5.1(9) leads to different types of product in the two cases. Thus, the $\lambda$Ag5.1(8) clone produces a fusion protein between $\beta$-galactosidase and a 7,700 daltons (by SDS-PAGE) P. falciparum protein whilst the $\lambda$Ag5.1(9) clone produces three P. falciparum proteins of molecular weight 22,000, 23,000 and 24,000 daltons (by SDS-PAGE), unfused to $\beta$-galactosidase. It seems likely that the proteins of molecular weight 22,000 and 23,000 daltons are products of the processing of the 24,000 daltons molecular weight protein by the E.coli. It is of interest to note in this context that, as discussed hereinbefore, that the 5.1-1 epitope-containing antigenic material isolated from nature having a molecular weight of 23,000 daltons was apparently converted on purification to a protein with a 22,000 daltons molecular weight. Moreover, the 5' terminus of the sequence of the structural gene present in clone $\lambda$Ag5.1(9) contains a set of 23 triplets which could be cleaved after the alanine residue at position 22 thereby forming a slightly smaller protein than that corresponding to that present in clone $\lambda$Ag5.1(9) gene.

As an alternative to the production of the antigenic materials described herein synthetically by genetic engineering techniques, materials corresponding to smaller peptides, in particular, may be prepared synthetically by chemical synthesis using standard techniques in the art of peptide synthesis.

The gentic engineering techniques described herein may of course be applied to the production of antigenic materials containing the whole or a part of an antigen of various strains of Plasmodium which occurs in nature in association with intra-erythrocytic forms of the parasite and carries an epitope which is shared with an antigenic material located on the sporozoite stage of the parasite, the present invention extending to products of the techniques as described herein for the KI Thailand P. falciparum strain derived by application of the techniques to such other strains.

The antigenic materials of the present invention are of particular value for incorporation into vaccines for inducing immunity to malaria in susceptible vertebrate hosts which are at risk of becoming infected by parasites of the genus Plasmodium. They are of especial interest for prophylactic use in man against P. falciparum. For use in vaccines the materials are preferably prepared in purified form, in particular it is preferred that they are in admixture with no more than 10%, and preferably no more than 1%, by weight, of other protein or peptide material. For many applications, it is especially desirable that the materials are substantially free from pyrogenic contaminants. In particular, in the case of materials isolated from nature, purification is conveniently effected to substantially remove materials of human or animal origin such as serum proteins, haemoglobin and blood group substances. In the case of materials which are hybrid proteins synthesised by genetically engineered clones of E. coli, purification is conveniently effected to substantially remove pyrogenic contaminanting bacterial proteins. Purification of such hybrid proteins may conveniently be facilitated by their reduced solubility in surface active agents such as Nonidet P40 (1%) permitting their separation as a precipitate from the majority of bacterial proteins. The precipitate may then be dissolved in the surface active agent sodium dodecylsulphate (5%) and the purity of the material confirmed in the case of proteins by SDS-PAGE and in the case of peptides by high performance liquid chromatography similar methods of confirming purity may also be applied to other antigenic materials within the scope of the present invention). It is generally the case that the antigenic materials, whether isolated from nature or otherwise are preferred to have a degree of purity which at least corresponds to their representing the major protein band on SDS-PAGE or peptide peak on h.p.l.c. and which conveniently corresponds to their being substantially free from other such protein or peptide bands or peaks.

Whilst it is preferred that the antigenic materials according to the present invention are used in the vaccines in a form substantially free from by-products of their preparation, it will be appreciated that the materials may be incorporated into the vaccines either singly or in combination either together with another such antigenic material or with a different antigenic material of value in such a context. In particular, it may be advantageous to include in a single the vaccine antigenic materials which on administration in vivo will provide antibodies and/or induce cellular immunity against various strains of one species of Plasmodium and/or against various species thereof. Moreover, it is generally preferred that the vaccine produces an immune response, conferring protective immunity, against various stages of the parasite's life cycle and, although the antigenic materials of the present invention are of particular utility in this respect, it may still be worthwhile including additional materials for this purpose.

The present invention thus includes a pharmaceutical composition which comprises an antigenic material suitable for use in vaccination against malaria ass defined hereinbefore together with a physiologically acceptable diluent or carrier. The diluents or carriers of particular interest are liquid media, a particular example being saline solution which may conveninetly be sterile and pyrogen free. The antigenic material may be in solution or suspension or may be solubilised by the addition of a physiologically acceptable detergent. The vaccine may, if desired, comprise some form of adjuvant for stimulating the immune response to the antigenic material, examples of adjuvant material which may be included as a separate component of the vaccine being saponin, Corynebacterium parvum (Coparvax) and aluminium hydroxide. An alternative way of stimulating the immune response is for the antigenic material to incorporate two parts, one of which possesses the antibody producing properties and the other of which enhances such production. Thus, if the antibody inducing substance is of relatively low molecular weight it may be appropriate as discussed hereinbefore to attach it to a carrier protein or other material in order to stimulate its immunogenicity. Alternatively, it may be possible to use antibodies of certain isotypes to improve immunogenicity of the antigenic material as described by Harte et al, Nature, 1983, 302, 256. In addition, attachment of antigenic substances to MHC antigens, as described in UK Patent 2004744, is a means of enhancing immunogenicity.

Conveniently the vaccines are formulated to contain a final concentration of antigenic protein in the range from 0.2 to 5 mg/ml, preferably 0.5 to 2 mg/ml, for example 1 mg/ml. After formulation, conveniently in unit dosage form (i.e. in the form of discrete portions containing a unit dose, or a multiple or sub-unit dose) the vaccine may be incorporated into a sterile container which is then sealed and stored at a low temperature, for example 4° C, or may be freeze dried. The doses envisaged in vertebrate hosts are at a level of 0.1 to 2 ml, preferably 0.2 to 1 ml, for example 0.5 ml, of vaccine containing the quantities of antigenic protein indicated previously. The vaccines may be administered by any convenient route although parenteral (for example subcutaneous or intramuscular injection) administration is of particular value, conveniently employing sterile, pyrogen-free formulations. The treatment may consist of a single dose of vaccine or a plurality of doses administered over a period of time.

The present invention thus includes an antigenic material suitable for use in vaccination against malaria as defined hereinbefore, for use in therapy.

The invention is illustrated by the following Examples.

## EXAMPLES

Example 1:

Development of a hybridoma cell line producing monoclonal antibodies reactive with the epitope 5.1-1

The procedure used is a modification of the protocol of Perrin et al., Nature, 1981, 289, 301. A P. falciparum isolate designated K1 from Thailand (Thaithong and Beale, Transactions of the Royal Society of Tropical Medicine and Hygiene, 1981, 75, 271) was grown in vitro according to the procedure of Trager and Jensen, Science, 1976, 193, 673 to give an asynchronous culture containing all asexual stages of the blood cycle, viz. ring forms, trophozoites, schizonts and merozoites.

Female Balb/c mice are immunized intraperitoneally with organisms released from host red cells, using a dose from 100 to 200 μg of total protein incorporated in incomplete Freunds's adjuvant given on 2-4 occasions separated by intervals of 2 weeks. The parasites are obtained by the use of a modification of the saponin treatment of Zuckerman et al., W.H.O. Bulletin, 1967, 37, 431, the infected erythrocytes being lysed with 0.1% saponin during 15 minutes at room temperature. This procedure gives preparations containing free parasites, parasites trapped in erythrocyte ghosts and red cell membranes, and is relatively free of haemoglobin which is removed by washing with serum-free culture medium.

At 2 to 16 weeks after the last intraperitoneal injection, the mice are injected intravenously with a similar preparation of parasites but in saline without adjuvant, a dose of 100 Σg of protein being used. Three days later the mice are killed and spleen cells obtained therefrom are fused with NS-1/1-Ag4-1 (NS-1) myeloma cells (Kohler et al., European Journal of Immunology, 1976, 6, 292) at ratios of 1:1 to 10:1 using the procedure of Oi and Herzenberg, Selected Methods in Cellular Immunology, 1980, 351 (edited by Mishell and Shiigi and published by Freeman, San Francisco). Two weeks after the fusion, those hybridoma cultures producing antibodies to plasmodia are identified by an immunofluorescence assay (IFA) according to the procedure of Voller and O'Neill, W.H.O. Bulletin, 1971, 45, 524, using acetone-fixed smears of infected blood.

Using the above described procedure a set of five separate fusions led to a total of 590 hybrid cultures of which 137 secreted antiplasmodial antibodies as detected by IFA. From these 137 hybrid cultures, 22 selected hybridomas were successfully cloned using limiting-dilution cloning with the addition of syngeneic thymocytes as feeder cells (1-2 x $10^5$ per microculture in 0.2 ml). The cultures were examined microscopically for the presence of hybridoma colonies ten days later, and supernatants from cultures containing only a single cluster of cells were screened for the presence of antimalarial antibody. Usually, 4-6 clones per primary hybridoma line were selected and expanded through passages firstly into 1 ml cluster-tray cultures and later in flask cultures (10 ml). The culture medium from each cloned hybridoma cell line was studied by IFA, various types of reactivity pattern being identified. From the 22 cell lines, one hybridoma cell line was selected on the basis of the strong reaction shown by its culture medium to a sporozoite preparation and was designated by the number 5.1-1, having been obtained by cloning of the hybrid culture 5.1 [1].

The hybridoma cell line 5.1-1 was maintained in culture at 37°C in a medium consisting of RPMI 1640 (supplied by Gibco) containing 10-15% v/v of foetal calf serum (FCS - supplied by Seralab Laboratories), with supplementation by sodium pyruvate to 1mM, glutamine to 2mM, penicillin to 500 units/ml and streptomycin to 500 mcg/ml (supplements also supplied by Gibco) and with the addition of sodium bicarbonate at a level of 0.2% w/v to produce a pH of 7.4. The cells were maintained in static cell culture at a density of 2-6 x $10^5$ cells/ml by dilution into fresh medium every 24-72 hours, being harvested once sufficient cells were generated. The atmosphere above the culture was maintained at 5-6% $CO_2$ in air with 85-95% relative humidity.

The cell line 5.1-1 has proved stable to growth by passaging in static culture for a period of at least two months but it may be desirable to reclone it periodically if growth in tissue culture is to be maintained for a prolonged period [2]. The cells deposited in the culture collection had been cloned once by limiting dilution into microcultures containing 0.2 ml of medium and $10^5$ Balb/c thymocytes as feeder cells. For the preparation of samples for storage (and of those for deposit), a suspension of the cells in culture medium is centrifuged at 250 g for 5 minutes. The cell pellet is suspended at room temperature in a freezing mixture which consists of 1 volume DMSO, 4 volumes of FCS and 5 volume of RPMI 1640, with a cell concentration not exceeding about 2 x $10^6$/ml. Cell aliquots to be frozen are closed in 2 ml plastic vials which are placed

[1] This hybrid culture derived from a fusion which utilised spleen cells from a mouse injected with saponin freed blood parasites administered in 4 intraperitoneal doses of 100-200 μg protein in Freund's incomplete adjuvant, followed by intravenous injection of schizonts (100 μg protein) 9 weeks after the last intraperitoneal injection. This fusion gave a set of cultures of which 50% contained viable hybrids but only two of these produced anti-malaria antibodies.

[2] 5.1-1 hybridoma cells present in ascitic fluid produced as described in Example 2 can alternatively be passaged in vivo in Balb/c mice.

in a small (about 15 x 15 x 15 cm³) well insulated polystyrene box. This is kept at -20°C for 3 hours and at -60°C overnight to allow slow cooling down and freezing of the cells. The vials are then transferred to liquid nitrogen for storage. (An alternative freezing mixture consists of 90% v/v FCS-10% v/v dimethyl sulphoxide at a density of 1-5 x 10⁶ cells/ml.)

To recover the cryopreserved cells, an aliquot is rapidly thawed in a 37°C incubator, the cells are washed in about 10 ml of serum-free medium and then set up in the RPMI 1640 medium with 20% FCS at a concentration of about 5 x 10⁵ cells/ml (the addition of thymocyte feeders at this stage is beneficial). When the cells begin to divide well, typically after 1-2 days, the culture is maintained and/or expanded as described previously.

Example 2:

Production of monoclonal antibody from the hybridoma cell line 5.1-1

(1) In vivo

5.1-1 hybridoma cells produced in tissue culture as described in Example 1 are grown as tumours by intraperitoneal inoculation into Balb/c mice. Each mouse receives about 3 x 10⁶ washed cells in the culture medium described in Example 1 (but lacking serum) after an initial priming 2-7 days previously with 0.5 ml pristane (2,6,10,14-tetramethylpentadecane; Aldrich Chemical Co.). Ascitic fluid is collected daily once the tumour develops. Antibody is prepared from the ascitic fluid by precipitation using 50% saturated ammonium sulphate, followed by ion exchange chromatography on diethyl amino ethyl cellulose (Whatman DE52) with an exponential convex gradient between the buffers of 0.005M sodium phosphate (pH 8.0) and 0.18M sodium phosphate (pH 8.0).

(2) In vitro

5.1-1 cells produced in tissue culture as described in Example 1 are cultured in a similar RPMI 1640-based medium as described in that Example but containing 10 % v/v of FCS, the culture being carried out in tissue culture flasks containing 50 ml of medium. When the medium above the cells turns acid, the cells are discarded and the supernatant harvested to prepare antibody by an analogous procedure to that described under (1) above involving $(NH_4)_2SO_4$ precipitation and DE52 chromatography.

Example 3:

**Distribution of 5.1-1 epitope-containing antigen in blood forms of different strains of P. falciparum**

The monoclonal antibody produced by the hybridoma 5.1-1 was tested according to the procedure described by McBride et al., Science, 1982, 217, 254, against a wide range of P. falciparum samples from different regions including both culture-adapted isolates (including clones) obtained from the WHO Reference Bank of Malaria Strains, Department of Genetics, University of Edinburgh, Scotland, and clinical samples obtained directly from human patients using the method described by McBride et al., Transactions of the Royal Society of Tropical Medicine and Hygiene, 1984. Each sample was tested by IFA (Voller and O'Neill, ibid) at least twice using parasites cultured according to the procedure of Trager and Jensen. ibid, harvested at weekly invervals; the homologous isolate K1, which was grown in parallel, being included as control. For testing, infected blood from cultures was washed in RPMI 1640 medium or saline and was then diluted in the same medium to provide 10⁴ to 10⁵ schizonts (and 10⁵ to 10⁷ of other stages) per 20 $\mu$l portion, each portion being dried onto the well of a multispot glass slide. The hybridoma culture fluid obtained as in (2) above was used undiluted at 80 fold excess of the concentration of antibody that still gave optimal reactions with the homologous isolate K1. In addition, a high titred ascitic fluid containing antibody 5.1-1 was tested at serial dilutions from 1 in 10 to 1 in 10⁶ on some samples. Background fluorescence was minimised by the use of a fluorescein isothiocyanate conjugate of immunoadsorbent-purified rabbit antiserum to mouse immunoglobulin (Miles-Yeda) and by counterstaining with Evans blue [0.1% in phosphate buffered saline (PBS)]. The stained slides were mounted in 50% glycerol in PBS and the reactions were scored under x500 magnification. Provided that any parasites at the appropriate stage of development (pigmented trophozoites and schizonts) reacted, the sample was scored as positive (although some samples clearly contained only a minority of positive organisms). Samples in which no parasites stained above the background were scored as negative.

The monoclonal antibody produced by the hybridoma 5.1-1 shows selective reactivity against most, but not all, of a wide range of P. falciparum isolates from different regions and against a majority of the wild strains tested. The results are shown in Tables 1 and 2; the isolates included in Table 1 are representative of the range in that they cover a range of endemic areas and are all samples which appear to be homogeneous and phenotypically stable, rather than containing a mixture of different antigenic types.

TABLE 1:

Reactivity of 5.1-1 epitope-specific antibody with culture-adapted

isolates of P. falciparum

| Isolate/clone | Origin | Reaction (culture fluid) | Reciprocal titre (ascitic fluid) |
|---|---|---|---|
| K 1 | Thailand | + | $10^4$ |
| K 28 | Thailand | + | not tested |
| SK 18 | Thailand | + | not tested |
| S 2 | Thailand | + | not tested |
| T9/clone94 | Thailand | + | $10^4$ |
| T9/clone 96 | Thailand | + | $10^4$ |
| T9/clone 105 | Thailand | + | not tested |
| SL 3 | Sri Lanka | + | not tested |
| MAD 20 | Papua New Guinea | + | $10^4$ |
| MAD 21 | Papua New Guinea | + | not tested |
| M 23 | Honduras | + | not tested |
| It | Brazil | + | not tested |
| RFCR-3 | Gambia | + | not tested |
| BW | Gambia | + | not tested |
| G1 | Gambia | + | $10^4$ |
| TZ | Tanzania | + | $10^4$ |
| K34 | Thailand | – | not tested |
| T9/clone 101 | Thailand | – | not tested |
| T9/clone 102 | Thailand | – | not tested |
| PB1 | Thailand | – | <10 |
| H1 | Honduras | – | <10 |
| Palo Alto | Uganda | – | <10 |

TABLE 2:

Reactivity of 5.1-1 epitope-specific antibody with wild strains

of P. falciparum

| Origin | Total number examined | Number positive | Number negative |
|---|---|---|---|
| Papua New Guinea | 109 | 100 | 9 |
| Gambia | 98 | 58 | 40 |
| Malawi | 1 | 0 | 1 |
| Zimbabwe | 1 | 0 | 1 |

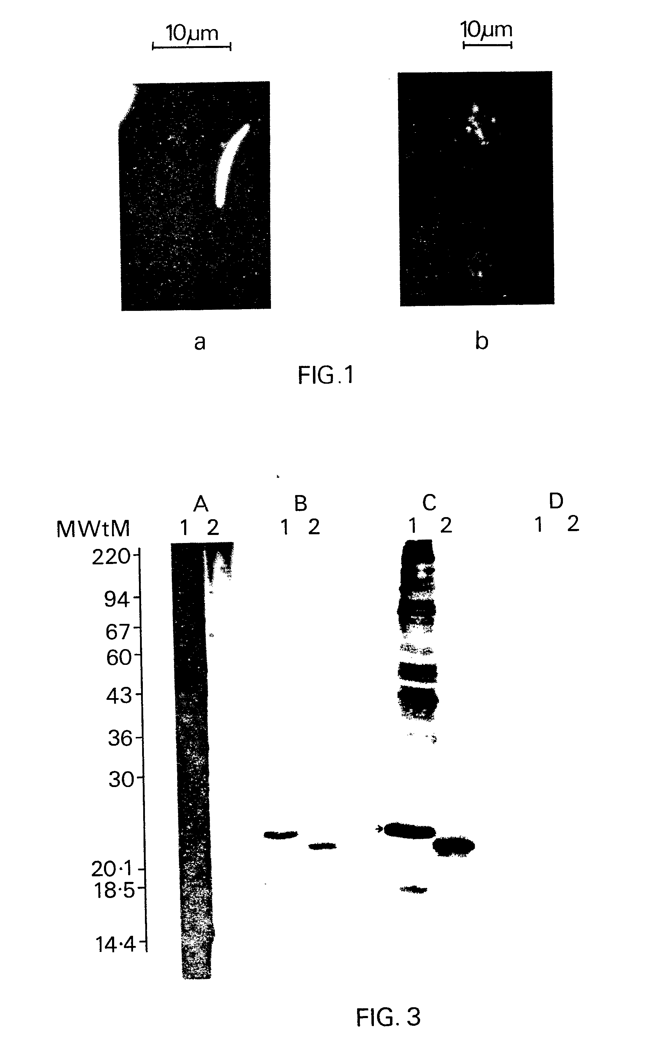

The indirect immunofluorescence staining of P. falciparum microscope preparations was also used to compare the behaviour of the monoclonal antibodies produced by the hybridoma 5.1-1 with glutaraldehyde fixed sporozoites collected in Thailand and air dried thin blood smears of an asynchronous in vitro culture, prepared according to the procedure Trager and Jensen, ibid, of P. falciparum, isolate K1, the parasitaemia being 5% and the slides being fixed in acetone for 5 minutes. The staining was carried out at room temperature in a moist chamber according to the procedure of Hall et al., Molecular biochemical parasitology, 1983, 7 247. The slides were incubated with the monoclonal antibodies for 30 minutes, then stained with the same Miles-Yeda conjugate as described above and again counterstained with Evans blue. The slides were examined by fluorescence microscopy after mounting in 50% glycerol. The results obtained are shown in Figure 1 which shows photographs taken at x1,000 magnification using Kodak Tri-X Pan film of (a) the sporozoites and (b) the blood smear. The bound antibody, which appears as a vivid green fluorescence is clearly seen in each case. The titre of the monoclonal antibody is high (1 in $10^6$ dilution) and it is equally active on sporozoites and erythrocytic stages. (Other monoclonal antibodies raised against erythrocytic stages which have been tested do not stain sporozoites even at a dilution of only 1 in 100).

Example 4:

Identification of protein occurring in vivo and containing the

epitope 5.1-1

An asynchronous in vitro culture of P. falciparum, isolate K1, (5% parasitaemia) is prepared according to the procedure of Trager and Jensen, ibid, washed once in PBS and lysed in 0.1% saponin in PBS at 4°C. The released parasites (approximately $2.5 \times 10^8$) are washed 3x in PBS before pelleting and freezing at -70°C for storage until required. The pellet is solubilised for electrophoresis by boiling in gel sample buffer and is then subjected to sodium dodecyl sulphate-polyacrylamide gel electrophoresis (SDS-PAGE) on 10% gels according to the procedure of Laemmli, Nature, 1970, 227, 690, the loading being approximately 500 μg of total protein. This amount of protein will overload the gel but this is done deliberately since the protein to be detected constitutes a minor proportion of the total proteins.

The proteins separated in the electrophoresis gel are transferred to nitrocellulose paper using the procedure known as Western blotting described by Towbin et al., Proceedings of the National Academy of Sciences of the USA, 1979, 76, 4350. The position of the desired protein on the nitrocellulose paper is revealed by the ELISA procedure as described by Towbin, ibid. The nitrocellulose blot is initially probed with 5.1-1 monoclonal antibody in the form of ascites fluid, prepared as described in Example 2, at a 1 in 200 dilution. This probe is followed by a polyvalent rabbit anti-mouse IgG, in the form of serum at a 1 in 500 dilution, and finally by horseradish peroxidase conjugated goat anti-rabbit IgG (Sigma) at a 1 in 1000 dilution. The conjugated enzyme is reacted with 0-dianisdine dihydrochloride (Sigma) and hydrogen peroxide giving a grown colour at the position of the desired protein.

For ease of comparison of the protein occurring in vivo with the other proteins containing the epitope binding to the monoclonal antibody 5.1-1 produced by the procedures of Examples 5 and 6 the characterising data on all these proteins in presented separately in Example 7.

Example 5:

Preparation of a purified protein containing the epitope 5.1-1

An affinity chromatography column is prepared by binding the monoclonal antibody 5.1-1, purified from ascites fluid by ammonium sulphate precipitation and DEAE ion exchange chromatography, to CNBr-activated Sepharose 4B (Pharmacia) according to the procedure prescribed by the manufacturer. This procedure involves reactivation of the CNBr-activated Sepharose 4B by washing in 1mM HCl. The immunoglobulin in 0.1M NaHCO$_3$, 0.5M NaCl is then contacted under rotation with the Sepharose material for 2 hours to effect binding therewith. The remaining binding sites on the material are inactivated by treatment thereof under rotation for 2 hours with 0.2M glycine of pH 8.0, followed by washing 3x in sequence with 0.1M acetate, 0.5M NaCl of pH 4.0 and 0.1M NaHCO$_3$, 0.5M NaCl. The resulting affinity chromatography material is stored in PBS.

Protein extracts from asynchronous cultures of P. falciparum containing 5 x 10$^{10}$ parasites, solubilised with the surfactant NP40 (BDH), were prepared as described by Holder and Freeman, Nature, 1981, 294, 361. The extracts were applied to the affinity chromatography column, which was washed briefly with the extraction buffer before elution of the bound protein with 8M urea at 4° C, a yield of approximately 10 $\mu$g of protein being obtained.

To produce a solid product, the eluate is dialysed against Tris-buffered physiological saline (ph 7.4), followed by concentration by lyophilisation. Characterising data on the protein of this Example is provided in Example 7.

Example 6:

Preparation of protein containing epitope 5.1-1 using mRNA

Proteins are synthesised in a reticulocyte lysate in vitro translation system (Pelham and Jackson, European Journal of Biochemistry, 1976, 67, 247) supplemented with $^{35}$S-methionine and P. facliparum polyA$^+$ mRNA from asynchronous cultures (Hyde et al., ibid).

A portion of the translated material (equivalent to 5 x 10$^6$ cpm) is treated on a 5.1-1 monoclonal antibody affinity chromatography column as described in Example 5. Characterising data on the protein of this Example is provided in Example 7.

Example 7:

Characterisation of various proteins containing epitope 5.1-1

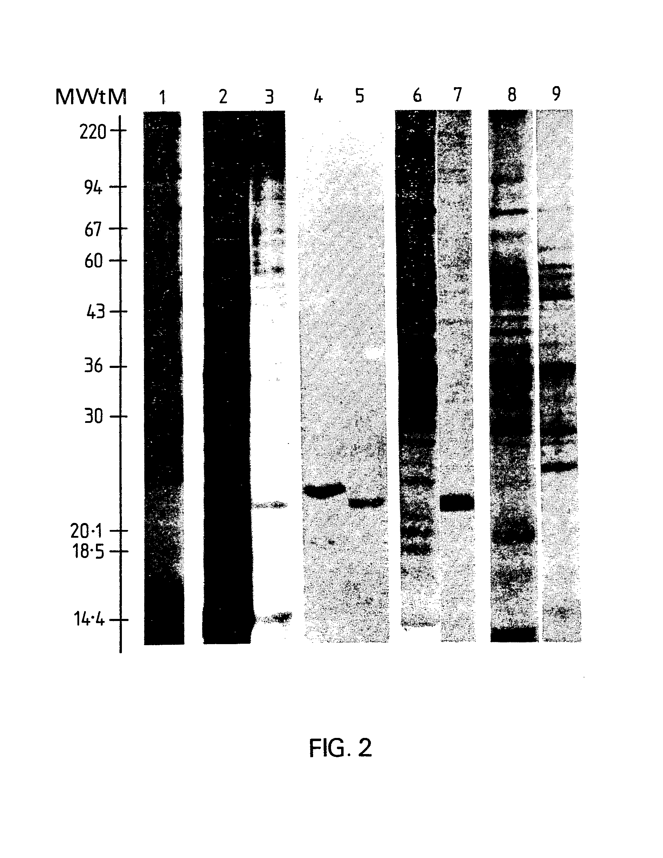

Figure 2 shows nine tracks typical of those obtained characterising the proteins of Examples 4, 5 and 6, which proteins are found to have molecular weight estimated at 23,000 daltons, 22,000 daltons and 24,500 daltons, respectively.

The various tracks are obtained as follows:

1. SDS-PAGE on total parasite proteins obtained as described in Example 4 and stained with Coomassie Brilliant Blue R.

2. As 2 but visualised by silver staining (Morrissey, Analytical Biochemistry, 1981, 117. 307).

3. SDS-PAGE by the procedure described in Example 4 on the protein obtained by affinity chromatography in Example 5; visualisation by silver staining.

4. Western blotting of total parasite proteins obtained and visualised as described in Example 4.

5. Western blotting of the SDS-PAGE track of the purified protein of Example 5, obtained and visualised as described in Example 4.

6. SDS-PAGE by Example 4 procedure on $^{35}$S-methionine labelled total parasite proteins [proteins labelled metabolically with $^{35}$S-methionine in an asynchronous culture according to Trager and Jensen, ibid, of P. falciparum by the method of Deans et al., Molecular and Biochemical Parasitology, 1983, 8, 45, and extracted with NP40 as described in Example 5 - contains 3,000 cpm of trichloroacetic acid (TCA) precipitable material]; the gel is processed for fluorography (Bonner and Laskey, European Journal of Biochemistry, 1974, 46, 83), dried and autoradiographed.

7. SDS-PAGE by Example 4 procedure on $^{35}$S-methionine labelled protein obtained by affinity chromatography of total proteins by procedure of Example 5 (contains $3 \times 10^6$ cpm of TCA precipitable material); the gel is processed for fluorography, dried and autoradiographed.

8. SDS-PAGE by Example 4 procedure on $^{35}$S-methionine labelled total in vitro translation products obtained as described in Example 6 (contains 5,000 cpm of TCA precipitable material); the gel is process for fluorography, dried and autoradiographed.

9. SDS-PAGE by Example 4 procedure on $^{35}$S-methionine labelled proteins separated from the total in vitro translation products by affinity chromatography as described in Example 6 ($5 \times 10^6$ cpm of TCA precipitable material); the gel is processed for fluorography, dried and autoradiographed.

The following molecular weight markers (MWtM, Pharmacia) are used to provide the scale shown in Figure 2: ferritin (220,000), phosphorylase b (94,000), albumin (67,000), catalase (60,000), ovalbumin (43,000), lactate dehydrogenase (36,000), carbonic anhydrase (30,000), trypsin inhibitor (20,100), ferritin (18,500) and $\alpha$-lactalbumin (14,400).

It will be seen in Figure 2 that in track 4 derived from total protein a single band carries the 5.1-1 epitope and that this band migrates as a protein with an estimated molecular weight of 23,000 daltons. Tracks 3 and 5 also show a single band carrying the epitope but in these instances the protein, which is the affinity chromatography purified material, migrates slightly faster at a rate corresponding to an estimated molecular weight of 22,000 daltons. Track 9 derived from chromatographed $^{35}$S-methioninelabelled mRNA total translated protein shows that the affinity chromatography column traps several of the in vitro produced products non-specifically. However, the column retains specifically a unique protein of estimated molecular weight 24,500 daltons.

Example 8:

Recognition by human sera of protein containing epitope 5.1-1

Figure 3 shows six tracks typical of those obtained characterising the reaction of the proteins of Examples 4 and 5 (estimated molecular weight 23,000 daltons and 22,000 daltons, respectively) with human sera. The tracks are obtained as follows:

A. SDS-PAGE obtained as in Example 7 on (1) the total parasite protein, being overloaded to enable antigens to be seen after Western blotting, and on (2) the protein obtained by affinity chromatography in Example 5; both gels being visualised by silver staining.

B. Western blotting of the proteins (1) and (2) of A transferred to nitrocellulose; probing by the ELISA procedure as in Example 7, the blot being probed first with 5.1-1 monoclonal antibodies in the form of ascites fluid, prepared as described in Example 2, at a 1 in 200 dilution and then with polyvalent rabbit anti-mouse IgG (1 in 500 dilution) followed by horseradish peroxidase conjugated goat anti-rabbit IgG (1 in 1000 dilution).

C. Western blotting of the proteins (1) and (2) of A transferred to nitrocellulose; probing by the ELISA procedure as in Example 7, the blot being probed intially with human sera (1 in 200 dilution) obtained by pooling 50 serum samples from pregnant women living in Nigeria, an area endemic for malaria, followed by horseradish peroxidase conjugated goat anti-human IgG (Sigma, 1 in 1000 dilution).

D. Western blotting of the proteins (1) and (2) of A transferred to nitrocellulose; probing by the ELISA procedure as in Example 7, the blot being probed initially with human sera (1 in 200 dilution) from an individual never exposed to P. falciparum malaria, followed by horseradish peroxidase conjugated goat anti-human IgG (Sigma, 1 in 1000 dilution). The scale shown is provided by the use of the same molecular weight markers as in Example 7.

In Figure 3 the bands revealed by the monoclonal antibodies 5.1-1 (B) and by endemic sera (C) are specific. No bands are seen with the non-immune serum (D). In track B1 the 5.1-1 epitope is detected in the form of the estimated 23,000 dalton molecular weight protein whilst in track B2 it appears in the form of the estimated 22,000 dalton molecular weight protein. The pooled human sera detect many antigens among the total parasite proteins in C1 but there is one particularly strong band (arrowed) which a comparison of band positions and intensities in tracks 1 and 2 of B and C suggests to be the 23,000 dalton estimated molecular weight protein. (It is found that the affinity chromatography purifed protein attached to Sepharose will typically bind with as much as 1% of the antibodies in this pooled sera.)

Example 9:

Preparation of bacterial clones expressing protein containing the epitope 5.1-1

A library of P. falciparum cDNA (derived from a P. falciparum isolate designated K1 from Thailand, described by Thaithong and Beale, Transactions of the Royal Society of Tropical Medicine and Hygiene, 1981, 75, 271) in the phage λgt11-Amp3 (lac5, nin5, cI857, S100, amp$^R$) is prepared as described by Kemp et al, Proceedings of the National Academy of Sciences of the USA, 1983, 80, 3787, the single stranded cDNA copied from the mRNA (purified as described by Hyde et al, Molecular and Biochemical Parasitology, 1984, 10, 269) being converted to double stranded cDNA using reverse transcriptase, the cDNA then being cloned into the EcoRI site of the phage by means of linkers and packaged into λ capsids in vitro (Scalenghe et al, Chromosoma (Berlin), 1981, 82 205). The library is plated in unamplified form on the E.coli Y1090 [ΔlacU169, Δlon, araD139, strA, thi, supF [trpC22::Tn10] (pMC9(lacI$^+$, amp$^R$))] described by Young and Davis, Science, 1983, 222, 778.

For screening of the λgt11-Amp3 cDNA library a polyclonal antibody probe specific for the natural erythrocytic stage P. falciparum antigen recognised by the antibodies produced by the hybridoma 5.1-1 is prepared as follows. The naturally derived, 23,000 dalton molecular weight (by SDS-PAGE), antigen is prepared from a non-ionic detergent extract of P. falciparum erythrocytic stages by affinity chromatography using an affinity column of the antibodies as described in Example 5 of UK Patent Application No. 8404493 and covalently coupled to CNBr-activated Sepharose 4B in a procedure similar to the one described in that Example to provide an affinity column carrying the antigen. Total human immunoglobulin from individuals living in an area endemic for falciparum malaria is applied to the affinity column in a physiological buffer (10 mM Tris, 0.15M NaCl, pH 7.4). After thorough washing of the column, polyclonal human antibody specific to the antigen is elated in a low pH buffer (0.1M glycine, 0.15M NaCl, pH 2.5).

The polyclonal antibody is used to screen the λgt11-Amp3 cDNA library by a procedure analogous to that described by Young and Davis, ibid, except that the immunological probing utilises a horseradish peroxidase conjugated second antibody as described by Towbin et al, Proceedings of the National Academy of Sciences of USA, 1979, 76, 4350.

Following the identification by this screening procedure of lysogens containing recombinant phage of particular interest, the recombinant phage, and the corresponding DNA and protein are obtained as follows.

Phage Preparation

E.coli Y1090 is lysogenised with the recombinant phage. This is done by mixing equal numbers of phage and growing bacteria at 30°C in L-broth (Bacto Tryptone, 10 g; Difco Bacto Yeast Extract, 5 g; NaCl, 5 g; water to 1 litre, pH 7.2) and selecting the lysogens by using a phage (λcI$^-$) which kills uninfected surviving bacteria but does not kill the desired lysogens [3]. Confirmed lysogens are grown in L-broth at 30°C with shaking to an OD$_{650}$ of 0.45. The lysogen is then induced by shaking in a 42°C water bath for 25 minutes and subsequent transfer to a 37°C incubator to allow growth to continue with shaking. The OD$_{650}$ value is followed and complete lysis of the culture is typically found to occur approximately 90 minutes after commencing incubation. Chloroform is then added to the culture at a level of 2 ml/litre and the culture is centrifuged (8,500 g, 10 minutes, 4°C). The supernatant is decanted off and the phage pelleted from it by centrifugation (43,000 g, 3 hours, 4°C). The phage is then resuspended in buffer (KH$_2$PO$_4$, 3 g; Na$_2$HPO$_4$, 7 g; NaCl, 5 g; 0.1M aqueous solution of MgSO$_4$, 10 ml; 0.01M aqueous solution of CaCl$_2$, 10 ml 1% aqueous solution of gelatin, 1 ml; water to 1 litre) at a dilution level of 10 ml of buffer/750 ml original culture by shaking at 4°C. To the suspension of phage in the phage buffer is added caesium chloride (7.3 g/10 ml of resuspended phage) the phage then being banded by centrifugation (100,000 g, 26 hours, 4°C), collected and dialysed against Tris (10 mM)-EDTA (1 mM) at pH 8.0 (Tris-EDTA).

DNA Preparation

DNA is prepared from the purified phage as follows. The suspension of phage is mixed with an equal volume of phenol/chloroform (1:1 v/v) to remove proteinaceous material, the aqueous phase being collected,

---

[3]Alternatively lysogens may be selected which are resistant to ampicillin (50 µg/ml).

this extraction procedure being performed four times. The final aqueous layer is then extracted twice with ether and the residual layer containing the DNA is treated with proteinase K (0.5 mg/ml final concentration) in a buffer (pH 8.0) containing Tris (50 mM) and SDS (0.05% v/v) to denature and digest residual protein. The treated solution is again extracted once with phenol/chloroform (1:1 v/v) and twice with ether. Finally the DNA is precipitated with ethanol and redissolved in Tris-EDTA (pH 8.0).

Protein Preparation

The E.coli lysogen is grown in L-broth at 30°C with shaking to an $OD_{650}$ of 0.3. Expression from the $\beta$-galactosidase gene is induced by adding a 100 mM aqueous solution of isopropyl thio-3-D-galactosidase(IPTG), sterilised by filtration, to the culture to produce a final IPTG concentration of 1mM. The shaking of the culture at 30°C is continued for a further 2 hours and the cells are then pelleted (4,000 g, 5 minutes, 22°C). The pelleted cells are taken up in Laemmli SDS-sample buffer (Nature, 1970, 227, 680) to a volume of 3% of that of the original culture. The buffer preparation is then centrifuged (12,000 g, 5 minutes) to remove non-solubilized material and the supernatant is subjected, at a level of 25 l per track to SDS-PAGE an 10% gels (Laemmli, ibid).

Example 10:

Preparation of the clones and λAg5.1(8) and λAg5.9(9) and of the corresponding DNA and protein

The procedure described in Example 9 was utilised to obtain two particular clones in the form of E.coli lysogens, these being designated as λAg5.1(8) and λAg5.1(9), the latter having been deposited under the accession number NCIB 12016. The reaction of different antibody probes to λAg5.1(8) and λAg5.1(9) was studied, three sets of filters being used, corresponding to λAg5.1(8), λAg5.1(9) and λgt11-Amp3 (as a control), with each filter being prepared from a plate with approximately 150 plaques of the appropriate phage, plated on E. coli Y1090. One of each set of filters was probed with (a) rabbit anti-$\beta$-galactosidase antiserum, (b) endemic human sera immunoglobulin, (c) endemic human sera immunoglobulin depleted of 5.1-1 epitope-specific antibodies, (d) polyclonal human antibody specific for the 5.1-1 epitope, and (e) monoclonal antibody from the hybridoma 5.1-1. Each of the phage gave plaques recognised by the rabbit anti-$\beta$-galactosidase antiserum whilst the λAg5.1(8) and λAg5.1(9) plaques, but not the λgt11-Amp3 plaques, reacted with endemic human sera immunoglobulin (but not with such serum depleted of 5.1-1 epitope-specific antibodies). The polyclonal human antibody specific for the 5.1-1 epitope showed virtually no non-specific binding to proteins in λgt11-Amp3 whilst λAg5.1(8) and λAg5.1(9) gave plaques which were recognised by this probe. Finally, it was found that λAg5.1(8) and λAg5.1(9) plaques, but not λgt11-Amp3 plaques, were also recognised by the monoclonal antibody produced by the hybridoma 5.1-1.

The DNA inserts contained in these clones have been isolated as described, recloned into M13mp11 (Messing et al, Gene, 1982, 19, 269) and sequenced using the dideoxy chain termination method, the λAg5.9(8) insert measuring about 330 base pairs and the λAg5.1(9) insert measuring about 660 base pairs by SDS-PAGE, although the actual number of base pairs present is somewhat less than this in each case. The overall structure of the clones is shown in Figure 4, the detailed structures determined for the inserts are shown in Figure 5(a) for clone λAg5.1(8) and in Figure 5(b) for clone λAg5.1(9). In each case the nucleotides present between the two GAATTC EcoRI sites are shown in 5' to 3' sequence from the top left hand corner to the bottom right hand corner of the page with both nucleotides and amino acid residues being numbered, the former above the nucleotide in round brackets (with the first digit of the number being above the nucleotide to which the number relates) and the latter below the residue in square brackets, starting with C of the CGC codon and the arginine residue corresponding to this codon each as 1 in Figure 5(a) and with A of the ATG codon and the methionine residue corresponding to this codon each as 1 in Figure 5(a). In these Figures, and elsewhere in this specification, the conventional symbols are employed, i.e. A, C, G and T being used to indicate nucleotides containing the bases adenine, cytosine, guanine and thymine, respectively and the various amino acid residues being represented by symbols as follows:

| | |
|---|---|
| Alanine | = Ala |
| Arginine | = Arg |
| Asparagine | = Asn |
| Aspartic acid | = Asp |
| Glutamine | = Gln |
| Glutamic acid | = Glu |
| Glycine | = Gly |

Histidine = His
Isoleucine = Ile
Leucine = Leu
Lysine = Lys
Methionine = Met
Phenylalanine = Phe
Proline = Pro
Serine = Ser
Threonine = Thr
Tyrosine = Tyr
Valine = Val

The two cDNA inserts overlap towards the 3' ends thereof, as is apparent from a comparison of Figures 5(a) and 5(b). It will be seen that there is a sequence of 15 base pairs following the EcoRI site in the λAg5.1(8) clone which differs partially from the true sequence of the gene present in the clone λAg5.1(9). This occurs because the avian myeloblastosis virus reverse transcriptase which is used to prime synthesis of the second strand of cDNA uses a loop in the first strand to effect such priming, base pairing in the loop region (which is represented by the 15 base pairs referred to) not being exact. Both inserts have a poly-A tail [of 18 and 12 residues in λAg5.1(8) and λAg5.1(9), respectively]. As the sequence also contains a nucleotide grouping AATAA providing a potential polyadenylation signal AAUAA upstream from the poly-A tail, it may be presumed that both clones represent the 3' terminus of the mRNA for the naturally occurring structural gene.

At its 5' terminus the λAg5.1(8) insert has an open reading frame in phase with β-galactosidase causing fusion of the β-galactosidase and of the P. falciparum protein produced by this clone. In contrast, the λAg5.1(9) insert has a nonsense codon at its 5' end in phase with β-galactosidase so that this clone does not give a fusion peptide. Translation of the λAg5.1(9) clone is believed to be initiated in E.coli at the ATG codon (numbered as base pairs 1, 2 and 3), there being in its vicinity (at positions -1, -2, -3, -20, +4, +5, +6, +7, +11 and +12) nucleotides which commonly occur in bacterial initiation sites, although there is no obvious Shine-Dalgarno sequence present. It seems likely that the same initiation codon (or a near upstream neighbour) is used in the parasite. At the 5' terminus of the coding sequence (at positions 1 to 69) a set of 23 codons is present which codes for a perfect signal peptide which could be cleaved after alanine-22. This finding accords with the observations that the parasite does export the protein and that the primary translation product is apparently bigger than the final form of the protein. In addition, it supports the designation made above of the initiation codon used in the parasite. It should be noted that the long internal sequence of hydrophobic residues valine-80 to tyrosine-101), may act as a stop-transfer sequence during processing of the protein.

It is believed that the coding sequence present in clone λAg5.1(9) comprising nucleotides 1 to 486 probably corresponds to the whole of the coding sequence of the natural structural gene since it directs the synthesis of a one hundred and sixty-two amino acid residue protein of a close, if not identical, mobility to that of the natural protein. The sequence of 18 amino acid residues running from asparagine-120 to proline-137, Asn-Ala-Asn-Pro-Asp-Ala-Asp-(Ser-Glu-Ser)-Asn-Gly-Glu-Pro-Asn-Ala-Asp-Pro, is believed to form the intra-erythrocytic stage epitope which is shared with the surface of the sporozoites, the fifteen non-bracketed residues being those believed to be of particular importance

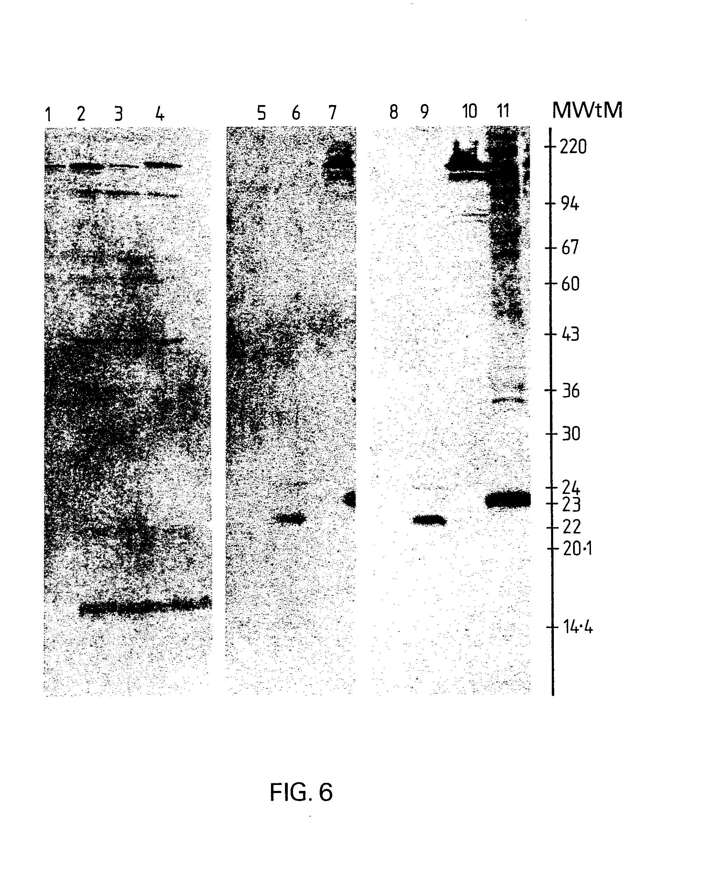

The proteins from the two clones λAg5.1(8) and λAg5.1(9) [4] were separated by SDS-PAGE according to the procedure of Laemmli referred to under "Protein Preparation" using the 10% w/v gels appropriate to the range of molecular weight which is of interest. The separated proteins were Western blotted to nitrocellulose and immunologically probed using horseradish peroxidase-conjugated second antibodies by the procedures of Towbin, ibid. The antibodies used were rabbit anti-β-galactosidase antiserum and the polyclonal human antibody specific for the natural antigen prepared as described hereinbefore at about a 1 in 200 dilution. These procedures are discussed in more detail in the Examples 4 and 7. The results obtained are shown in Figure 6 where tracks 1 to 4 relate to probing with rabbit anti-β-galactosidase antiserum and tracks 5 to 11

[4] In a variation of the procedures described in this Example lysogens of the two phage clones λAg5.1(8) and λAg5.1(9) are also prepared in the E.coli BTA282 [Δlac U169, Δlon, ara D139, str R, thi, hf1A 150 (chr::Tn10) hsdR M] described by Young and Davis, Proceedings of the National Academy of Sciences of the USA, 1983, 80, 7194, this strain lacking the lac repressor which E.coli Y1090 contains so that the gene cannot be switched. These E.coli BTA282 lysogens are prepared from purified phage λAg5.1(8) and λ5.1(9), obtained as described under "Phage Preparation", in an analogous fashion to that used for the preparation of the E.coli Y1090 lysogens.

relate to probing with the polyclonal human

The E.coli BTA282 lysogens are used to prepare proteins in an analogous manner to that described for the E.coli Y190 lysogens except that IPTG is not used, the lysogens instead being induced by raising the temperature to 42°C in order to increase the yield of protein in the absence of the control possible with IPTG in the other case. Although the yield of protein from the Y1090 lysogens is better, the BTA282 lysogens still give an acceptable yield. antibody. Track 1 contains $\beta$-galactosidase (0.2 g, SIGMA) whilst tracks 2 to 11 contain total protein from the following induced lysogens: E.coli BTA282 ($\lambda$gt11-Amp3) (tracks 2 and 5), E.coli BTA282 ($\lambda$Ag5.1(9)) tracks 3 and 6), E.coli BTA282 ($\lambda$Ag5.1(8)) (tracks 4 and 7), E.coli Y1090 ($\lambda$gt11-Amp3) (track 8), E.coli Y1090 ($\lambda$Ag5.1(9)) (track 9) and E.coli Y1090 ($\lambda$Ag5.1(8)) (track 10). Track 11 contains proteins from $2.5 \times 10^8$ saponin-lysed erythrocytic stage parasites (P. falciparum, isolate K1). The molecular weight markers used to provide the k.dalton MWtM scale are from Pharmacia as follows: ferritin (220,000), phosphorylase b (94,000), albumin (67,000), catalase (60,000), ovalbumin (43,000), lactate dehydrogenase (36,000), carbonic anhydrase (30,000), trypsin inhibitor (20,100), ferritin (18,500), and $\alpha$-lactalbumin (14,400). The figures of 22, 23 and 24 k.daltons on the scale are extrapolated from the 20.1 and 14.4 k.dalton markers, being shown for convenience.

It will be seen from Figure 6 that the $\lambda$Ag5.1(8) lysogens make a fusion protein which is larger than the native $\beta$-galactosidase sub-unit (compare tracks 1, 2 and 4) and which is recognised by the polyclonal human antibody probe (see tracks 7 and 10). By contrast, the control lysogen containing the vector alone makes a $\beta$-galactosidase sub-unit of normal size which is detected only by the anti-$\beta$-galactosidase antibody (see tracks 2 and 8). The $\lambda$Ag5.1(9) lysogens do not make a fusion protein since the expressed $\beta$-galactosidase sub-unit is of unchanged size (see track 3) and only binds with the antibody against the enzyme (see tracks 6 and 9). However, they do make proteins which bind the human polyclonal antibody, suggesting that in this clone translation of the protein containing the 5.1-1 epitope initiates independently. There are 3 bands of similar size (see tracks 6 and 9) and of these, one migrates with substantially the same mobility ($M_R$, 24,000 daltons) as the parasite's primary translation product. The others ($M_R$ 23,000 and 22,000 daltons) are believed to be forms of the primary product which have been processed in the bacterium, one of them ($M_R$ 22,000 daltons) having the same mobility as the purified parasite protein. Thus, the gene cloned in $\lambda$Ag5.1(9) is translated and possibly processed by E.coli.

## Claims

1. An antigenic material suitable for use in vaccination against malaria which (a) is an antigenic substance characterised by:

   (1) being an immunogenic proteinaceous antigen of a parasite of the species Plasmodium falciparum occurring in nature in association with intra-erythrocytic forms of the parasite;

   (2) having a molecular weight as determined by SDS-PAGE in the range of 20,000 to 25,000 daltons; and

   (3) comprising a sequence of amino acid residues containing an epitope, which epitope is shared with an antigenic material located on the sporozoite stage of the parasite so that on administration in vivo the antigenic substance generates antibodies reactive with both intra-erythrocytic forms of the parasite and with the sporozoite surface of that parasite;

   or (b) is an antigenic substance derivable from the antigenic substance defined in (a) and which contains a whole or a part of the sequence of amino acid residues therein including those residues which constitute said epitope so that on administration in vivo the antigenic substance generates antibodies reactive with both intra-erythrocytic forms of the parasite and with the sporozoite surface of that parasite.

2. An antigenic material according to Claim 1 having a molecular weight of 23,000 daltons.

3. An antigenic material according to Claim 1 having a molecular weight of 22,000 daltons.

4. An antigenic material according to any of Claims 1 to 3 which is in admixture with no more than 10% by weight of other protein or peptide material.

5. An antigenic material according to any of Claims 1 to 4 which is obtained synthetically.

6. An antigenic material according to Claim 5 which is obtained by genetic engineering.

EP 0 153 188 B1

7. An antigenic material according to any of Claims 1 to 6 attached to a carrier protein.

8. Recombinant DNA comprising a cloning vehicle sequence and DNA which comprises either the whole or a portion or portions of the following nucleotide sequence including a sequence coding for an epitope present in intra-erythrocytic forms of a parasite of the species Plasmodium falciparum which is shared with an antigenic material located on the sporozoite stage of the parasite:

|     |     |     |     |     |     |     |     |     |     |     |
|-----|-----|-----|-----|-----|-----|-----|-----|-----|-----|-----|
| ATG | AAA | ATC | TTA | TCA | GTA | TTT | TTT | CTT | GCT | CTT |
| TTC | TTT | ATC | ATT | TTC | AAT | AAA | GAA | TCC | TTA | GCC |
| GAA | AAA | ACA | AAC | AAA | GAA | ACT | GGA | AGT | GGT | GTT |
| AGC | AGC | AAA | AAA | AAA | AAT | AAA | AAA | GGA | TCA | GGT |
| GAA | CCA | TTA | ATA | GAT | GTA | CAC | GAT | TTA | ATA | TCT |
| GAT | ATG | ATC | AAA | AAA | GAA | GAA | GAA | CTT | GTT | GAA |
| GTT | AAC | AAA | AGA | AAA | TCC | AAA | TAT | AAA | CTT | GCC |
| ACT | TCA | GTA | CTT | GCA | GGT | TTA | TTA | GGT | GTA | GTA |
| TCC | ACC | GTA | TTA | TTA | GGA | GGT | GTT | GGT | TTA | GTA |
| TTA | TAC | AAT | ACT | GAA | AAA | GGA | AGA | CAC | CCA | TTC |
| AAA | ATA | GGA | TCA | AGC | GAC | CCA | GCT | GAT | AAT | GCT |
| AAC | CCA | GAT | GCT | GAT | TCT | GAA | TCC | AAT | GGA | GAA |
| CCA | AAT | GCA | GAC | CCA | CAA | GTT | ACA | GCT | CAA | GAT |
| GTT | ACA | CCA | GAG | CAA | CCA | CAA | GGT | GAC | GAC | AAC |
| AAC | CTC | GTA | AGT | GGC | CCT | GAA | CAC |     |     |     |

or which comprises an equivalent nucleotide sequence coding for the same amino acid residues.

9. Recombinant DNA according to Claim 8 which contains two linked nucleotide sequences AAT GCT AAC CCA GAT GCT GAT and AAT GGA GAA CCA AAT GCA GAC CCA or equivalent nucleotide sequences coding for the same amino acid residues.

10. Recombinant DNA according to Claim 9, in which the terminal GAT codon of the last sequence and the terminal AAT codon of the second sequence are joined by the nucleotide sequence TCT GAA TCC or an equivalent nucleotide sequence coding for the same three amino acid residues.

11. Recombinant DNA according to Claim 8 which contains the following nucleotide sequence:

|     |     |     |     |     |     |     |     |     |     |     |
|-----|-----|-----|-----|-----|-----|-----|-----|-----|-----|-----|
| CGC | AAG | TTT | ATA | TTT | GGA | GGT | GTT | GGT | TTA | GTA |
| TTA | TAC | AAT | ACT | GAA | AAA | GGA | AGA | CAC | CCA | TTC |
| AAA | ATA | GGA | TCA | AGC | GAC | CCA | GCT | GAT | AAT | GCT |
| AAC | CCA | GAT | GCT | GAT | TCT | GAA | TCC | AAT | GGA | GAA |
| CCA | AAT | GCA | GAC | CCA | CAA | GTT | ACA | GCT | CAA | GAT |
| GTT | ACA | CCA | GAG | CAA | CCA | CAA | GGT | GAC | GAC | AAC |
| AAC | CTC | GTA | AGT | GGC | CCT | GAA | CAC |     |     |     |

or an equivalent nucleotide sequence coding for the same amino acid residues.

12. Recombinant DNA comprising a cloning vehicle sequence and the P. falciparum derived nucleotide

27

sequence present in the Escherichia coli lysogen NCIB 12016.

13. Recombinant DNA according to any of Claims 8 to 12, in which the cloning vehicle sequence derives from a lambda phage.

14. Recombinant DNA according to Claim 13, in which the cloning vehicle sequence derives from phage λgt11 or a modificationn thereof.

15. The recombinant DNA contained in the clone λAg5.1(9) deposited as the Escherichia coli lysogen NCIB 12016.

16. Host cells comprising recombinant DNA according to any of Claims 8 to 15.

17. Host cells according to Claim 16 which are derived from Escherichia coli.

18. The Escherichia coli lysogen NCIB 12016 containing the clone λAg5.1(9).

19. A hybridoma cell line which produces an antibody which is reactive with both the antigenic material according to Claim 1 and the sporozoite surface of the parasite.

20. Antibodies produced by a hybridoma according to Claim 19.

21. Antibodies according to Claim 20 in the form of a composition comprising a physiologically acceptable diluent or carrier.

22. The use of antibodies according to Claim 20 in the preparation of antigenic materials according to any of Claims 1 to 7.

23. Antibodies according to Claim 20 in immobilised form.

24. A process for use in the preparation of an antigenic material according to any of Claims 1 to 7 which comprises absorbing said antigenic material or a precursor therefor on a solid support material which carries antibodies according to Claim 20 and thereafter eluting the antigenic material therefrom.

25. A pharmaceutical composition which comprises an antigenic material according to any of Claims 1 to 7 together with a physiologically acceptable diluent or carrier.

26. An antigenic material according to any of Claims 1 to 7 in the form of a vaccine suitable for use in vaccination against malaria.

27. An antigenic material according to any of Claims 1 to 7 for use in therapy.

**Revendications**

1. Produit antigénique approprié pour être utilisé dans la vaccination contre le paludisme, lequel produit (a) est une substance antigénique, caractérisée par le fait que :
(1) c'est un antigène protéiné immunogène d'un parasite de l'espèce Plasmodium falciparum survenant dans la nature en association avec les formes intra-érythrocytaires du parasite;
(2) elle a un poids moléculaire, comme déterminé par la procédure SDS-PAGE, dans la plage de 20 000 à 25 000 daltons; et
(3) elle comprend une séquence de résidus d'acides aminés contenant un épitope, lequel épitope est associé à un produit antigénique situé sur le stade sporozoïte du parasite, de telle sorte que, lors d'une administration in vivo, la substance antigénique produit des anticorps réagissant à la fois avec les formes intra-érythrocytaires du parasite et avec la surface des sporozoïtes de ce parasite;
ou (b) est une substance antigénique pouvant être dérivée de la substance antigénique définie en (a) et qui contient la totalité ou une partie de la séquence des résidus d'acides aminés, y compris ces résidus qui constituent cet épitope, de sorte que, lors d'une administration in vivo, la substance antigénique produit des anticorps réagissant à la fois avec les formes intra-érythrocytaires du parasite et avec la surface des sporozoïtes de ce parasite.

2. Produit antigénique selon la revendication 1, ayant un poids moléculaire de 23 000 daltons.

3. Produit antigénique selon la revendication 1, ayant un poids moléculaire de 22 000 daltons.

4. Produit antigénique selon l'une des revendications 1 à 3, qui est en mélange avec pas plus de 10% en poids d'une autre protéine ou d'un autre peptide.

5. Produit antigénique selon l'une des revendications 1 à 4, qui est obtenu par synthèse.

6. Produit antigénique selon la revendication 5, qui est obtenu par génie génétique.

7. Produit antigénique selon l'une des revendications 1 à 6, fixé sur une protéine support.

8. ADN recombinant, comportant une séquence de supports de clonage et un ADN qui comprend, soit la totalité, soit une portion ou des portions de la séquence de nucléotides suivante , contenant une séquence codant pour un épitope présent dans les formes intra-érythrocytaires d'un parasite de l'espèce Plasmodium falciparum, qui est associé à un produit antigénique situé sur le stade sporozoïte du parasite :

| ATG | AAA | ATC | TTA | TCA | GTA | TTT | TTT | CTT | GCT | CTT |
|-----|-----|-----|-----|-----|-----|-----|-----|-----|-----|-----|
| TTC | TTT | ATC | ATT | TTC | AAT | AAA | GAA | TCC | TTA | GCC |
| GAA | AAA | ACA | AAC | AAA | GAA | ACT | GGA | AGT | GGT | GTT |
| AGC | AGC | AAA | AAA | AAA | AAT | AAA | AAA | GGA | TCA | GGT |
| GAA | CCA | TTA | ATA | GAT | GTA | CAC | GAT | TTA | ATA | TCT |
| GAT | ATG | ATC | AAA | AAA | GAA | GAA | GAA | CTT | GTT | GAA |
| GTT | AAC | AAA | AGA | AAA | TCC | AAA | TAT | AAA | CTT | GCC |
| ACT | TCA | GTA | CTT | GCA | GGT | TTA | TTA | GGT | GTA | GTA |
| TCC | ACC | GTA | TTA | TTA | GGA | GGT | GTT | GGT | TTA | GTA |
| TTA | TAC | AAT | ACT | GAA | AAA | GGA | AGA | CAC | CCA | TTC |
| AAA | ATA | GGA | TCA | AGC | GAC | CCA | GCT | GAT | AAT | GCT |
| AAC | CCA | GAT | GCT | GAT | TCT | GAA | TCC | AAT | GGA | GAA |
| CCA | AAT | GCA | GAC | CCA | CAA | GTT | ACA | GCT | CAA | GAT |
| GTT | ACA | CCA | GAG | CAA | CCA | CAA | GGT | GAC | GAC | AAC |
| AAC | CTC | GTA | AGT | GGC | CCT | GAA | CAC | | | |

ou qui comprend une séquence de nucléotides équivalente codant pour les mêmes résidus d'acides aminés.

9. ADN recombinant selon la revendication 8, qui contient deux séquences de nucléotides liées AAT GCT AAC CCA GAT GCT GAT et AAT GGA GAA CCA AAT GCA GAC CCA ou des séquences de nucléotides équivalentes codant pour les mêmes résidus d'acides aminés.

10. ADN recombinant selon la revendication 9, dans lequel le codon terminal GAT de la première séquence et le codon terminal AAT de la deuxième séquence sont reliés par la séquence de nucléotides TCT GAA TCC ou par une séquence de nucléotides équivalentes codant pour les mêmes trois résidus d'acides aminés.

11. ADN recombinant selon la revendication 8, qui contient la séquence de nucléctides suivante :

| CGC | AAG | TTT | ATA | TTT | GGA | GGT | GTT | GGT | TTA | GTA |
|-----|-----|-----|-----|-----|-----|-----|-----|-----|-----|-----|
| TTA | TAC | AAT | ACT | GAA | AAA | GGA | AGA | CAC | CCA | TTC |
| AAA | ATA | GGA | TCA | AGC | GAC | CCA | GCT | GAT | AAT | GCT |
| AAC | CCA | GAT | GCT | GAT | TCT | GAA | TCC | AAT | GGA | GAA |
| CCA | AAT | GCA | GAC | CCA | CAA | GTT | ACA | GCT | CAA | GAT |
| GTT | ACA | CCA | GAG | CAA | CCA | CAA | GGT | GAC | GAC | AAC |
| AAC | CTC | GTA | AGT | GGC | CCT | GAA | CAC |     |     |     |

ou une séquence de nucléotides équivalente codant pour les mêmes résidus d'acides aminés.

12. ADN recombinant comprenant une séquence de supports de clonage et la séquence de nucléotides dérivée du P. falciparum présente dans le lysogène d'Escherichia coli NCIB 12016.

13. ADN recombinant selon l'une des revendications 8 à 12, dans lequel la séquence des supports de clonage dérive d'un phage lambda.

14. ADN recombinant selon la revendication 13, dans lequel la séquence de véhicules de clonage dérive du phage λgt11 ou d'une modification de celui-ci.

15. ADN recombinant contenu dans le clone λAg5.1(9) déposé en tant que lysogène de Escherichia coli NCIB 12016.

16. Cellules hôtes comprenant un ADN recombinant selon l'une des revendications 8 à 15.

17. Cellules hôtes selon la revendication 16, qui sont dérivées de Escherichia coli.

18. Lysogène de l'Escherichia coli NCIB 12016 contenant le clone λAg5.1(9).

19. Lignée de cellules hybridomes, qui produit un anticorps réagissant à la fois avec le produit antigénique selon la dication 1 et avec la surface des sporozoïtes du parasite.

20. Anticorps produits par un hybridome selon la revendication 19.

21. Anticorps selon la revendication 20, sous la forme d'une composition comprenant un diluant ou un support physiologiquement acceptable.

22. Utilisation d'anticorps selon la revendication 20, dans la préparation de produits antigéniques selon l'une des revendications 1 à 7.

23. Anticorps selon la revendication 20, sous forme immobilisée.

24. Procédé utilisé dans la préparation de produits antigéniques selon l'une des revendications 1 à 7, qui consiste à absorber ce produit antigénique ou un précurseur de celui-ci sur un support solide qui porte des anticorps selon la revendication 20 et à en éluer ensuite le produit antigénique.

25. Composition pharmaceutique, qui comprend un produit antigénique selon l'une des revendications 1 à 7 avec un diluant ou un support physiologiquement acceptable.

26. Produit antigénique selon l'une des revendications 1 à 7, sous la forme d'un vaccin approprié pour être utilisé dans la vaccination contre le paludisme.

**27.** Produit antigénique selon l'une des revendications 1 à 7, pour être utilisé dans une thérapie.

**Patentansprüche**

**1.** Antigenes Material, geeignet zur Verwendung bei der Impfung gegen Malaria, dadurch **gekennzeichnet,** daß es (a) eine antigene Substanz ist, die dadurch gekennzeichnet ist, daß sie

(1) ein immunogenes proteinartiges Antigen eines Parasiten der Spezies Plasmodium falciparum ist, das natürlich in Assoziation mit intra-erythrozytären Formen des Parasiten vorkommt;

(2) ein Molekulargewicht, bestimmt durch SDS-PAGE im Bereich von 20.000 bis 25.000 Dalton besitzt; und

(3) eine Aminosäuresequenz umfaßt, die ein Epitop enthält, das von einem antigenen Material, angeordnet auf dem Sporozoiten-Stadium des Parasiten, geteilt wird, so daß bei in vivo Verabreichung die antigene Substanz Antikörper erzeugt, die sowohl mit intra-erythrozytären Formen des Parasiten als auch mit der Sporozoiten-Oberfläche des Parasiten reagieren,

oder (b) eine antigene Substanz ist, die von der antigenen Substanz, definiert in (a), ableitbar ist und die eine ganze oder Teilsequenz von Aminosäureresten enthält, eingeschlossen darin die Reste, die das Epitop bilden, so daß bei in vivo Verabreichung die antigene Substanz Antikörper erzeugt, die sowohl mit den intra-erythrozytären Formen des Parasiten als auch mit der Sporozoiten-Oberfläche des Parasiten reagieren.

**2.** Antigenes Material nach Anspruch 1, dadurch **gekennzeichnet,** daß es ein Molekulargewicht von 23.000 Dalton besitzt.

**3.** Antigenes Material nach Anspruch 1, dadurch **gekennzeichnet,** daß es ein Molekulargewicht von 22.000 Dalton besitzt.

**4.** Antigenes Material nach einem der Ansprüche 1 bis 3, dadurch **gekennzeichnet,** daß es im Gemisch mit nicht mehr als 10 Gew.-% eines anderen Protein- oder Peptidmaterials vorliegt.

**5.** Antigenes Material nach einem der Ansprüche 1 bis 4, dadurch **gekennzeichnet,** daß es synthetisch erhalten worden ist.

**6.** Antigenes Material nach Anspruch 5, dadurch **gekennzeichnet,** daß es gentechnisch erhalten worden ist.

**7.** Antigenes Material nach einem der Ansprüche 1 bis 6, dadurch **gekennzeichnet,** daß es an ein Trägerprotein gebunden ist.

**8.** Rekombinante DNA, umfassend die Sequenz eines Klonierungsvehikels und eine DNA, dadurch **gekennzeichnet,** daß sie die folgende Nukleotidsequenz einschließlich einer Sequenz, die für ein Epitop codiert, das in intra-erythrozytären Formen eines Parasiten der Spezies Plasmodium falciparum vorkommt, das von einem antigenen Material, das auf dem Sporozoiten-Stadium des Parasiten angeordnet ist, geteilt wird, ganz oder in einem Teil oder in Teilen, umfaßt:

```
ATG   AAA   ATC   TTA   TCA   GTA   TTT   TTT   CTT   GCT   CTT
TTC   TTT   ATC   ATT   TTC   AAT   AAA   GAA   TCC   TTA   GCC
GAA   AAA   ACA   AAC   AAA   GAA   ACT   GGA   AGT   GGT   GTT
AGC   AGC   AAA   AAA   AAA   AAT   AAA   AAA   GGA   TCA   GGT
GAA   CCA   TTA   ATA   GAT   GTA   CAC   GAT   TTA   ATA   TCT
GAT   ATG   ATC   AAA   AAA   GAA   GAA   GAA   CTT   GTT   GAA
GTT   AAC   AAA   AGA   AAA   TCC   AAA   TAT   AAA   CTT   GCC
ACT   TCA   GTA   CTT   GCA   GGT   TTA   TTA   GGT   GTA   GTA
TCC   ACC   GTA   TTA   TTA   GGA   GGT   GTT   GGT   TTA   GTA
TTA   TAC   AAT   ACT   GAA   AAA   GGA   AGA   CAC   CCA   TTC
AAA   ATA   GGA   TCA   AGC   GAC   CCA   GCT   GAT   AAT   GCT
AAC   CCA   GAT   GCT   GAT   TCT   GAA   TCC   AAT   GGA   GAA
CCA   AAT   GCA   GAC   CCA   CAA   GTT   ACA   GCT   CAA   GAT
GTT   ACA   CCA   GAG   CAA   CCA   CAA   GGT   GAC   GAC   AAC
AAC   CTC   GTA   AGT   GGC   CCT   GAA   CAC
```

oder daß sie eine äquivalente Nukleotidsequenz, die für die gleichen Aminosäurereste codiert, umfaßt.

9. Rekombinante DNA nach Anspruch 8, dadurch **gekennzeichnet,** daß sie zwei verknüpfte Nukleotidsequenzen AAT GCT AAC CCA GAT GCT GAT und AAT GGA GAA CCA AAT GCA GAC CCA oder äquivalente Nukleotidsequenzen, die für die gleichen Aminosäurereste codieren, umfaßt.

10. Rekombinante DNA nach Anspruch 9, dadurch **gekennzeichnet,** daß das terminale GAT Codon der letzten Sequenz und das terminale AAT Codon der zweiten Sequenz durch die Nukleotidsequenz TCT GAA TCC oder eine äquivalente Nukleotidsequenz, die für die gleichen drei Aminosäurereste codiert, verknüpft sind.

11. Rekombinante DNA nach Anspruch 8, dadurch **gekennzeichnet,** daß sie die folgende Nukleotidsequenz

```
CGC   AAG   TTT   ATA   TTT   GGA   GGT   GTT   GGT   TTA   GTA
TTA   TAC   AAT   ACT   GAA   AAA   GGA   AGA   CAC   CCA   TTC
AAA   ATA   GGA   TCA   AGC   GAC   CCA   GCT   GAT   AAT   GCT
AAC   CCA   GAT   GCT   GAT   TCT   GAA   TCC   AAT   GGA   GAA
CCA   AAT   GCA   GAC   CCA   CAA   GTT   ACA   GCT   CAA   GAT
GTT   ACA   CCA   GAG   CAA   CCA   CAA   GGT   GAC   GAC   AAC
AAC   CTC   GTA   AGT   GGC   CCT   GAA   CAC
```

oder eine äquivalente Nukleotidsequenz, die für die gleichen Aminosäurereste codiert, umfaßt.

12. Rekombinante DNA, dadurch **gekennzeichnet,** daß sie die Sequenz eines Klonierungsvehikels und die von P. falciparum abgeleitete Nukleotidsequenz, die in dem Escherichia coli-Lysogen NCIB 12016 vorhanden ist, umfaßt.

13. Rekombinante DNA nach einem der Ansprüche 8 bis 12, dadurch **gekennzeichnet,** daß sich die Sequenz des Klonierungsvehikels von einem Lambdaphagen ableitet.

**14.** Rekombinante DNA nach Anspruch 13, dadurch **gekennzeichnet,** daß sich die Sequenz des Klonierungsvehikels von dem Phagen λgt11 oder einer Modifikation davon ableitet.

**15.** Rekombinante DNA, enthalten in dem Klon λAg5.1(9), hinterlegt als <u>Escherichia coli</u>-Lysogen NCIB 12016.

**16.** Wirtszellen, dadurch **gekennzeichnet,** daß sie eine rekombinante DNA nach einem der Ansprüche 8 bis 15 umfassen.

**17.** Wirtszellen nach Anspruch 16, dadurch **gekennzeichnet,** daß sie sich von <u>Escherichia coli</u> ableiten.

**18.** <u>Escherichia coli</u>-Lysogen NCIB 12016, enthaltend den Klon λAg5.1(9).

**19.** Hybridoma-Zellinie, dadurch **gekennzeichnet,** daß sie einen Antikörper produziert, der sowohl mit dem antigenen Material nach Anspruch 1, als auch der Sporozoiten-Oberfläche des Parasiten reagiert.

**20.** Antikörper, gebildet von einem Hybridom nach Anspruch 19.

**21.** Antikörper nach Anspruch 20, dadurch **gekennzeichnet,** daß sie in Form eines Präparats, umfassend einen physiologisch annehmbaren Diluenten oder Träger, vorliegen.

**22.** Verwendung der Antikörper nach Anspruch 20 zur Herstellung von antigenen Materialien nach einem der Ansprüche 1 bis 7.

**23.** Antikörper nach Anspruch 20 in immobilisierter Form.

**24.** Verfahren zur Verwendung bei der Herstellung eines antigenen Materials nach einem der Ansprüche 1 bis 7, dadurch **gekennzeichnet,** daß man das antigene Material oder einen Vorläufer davon auf einem festen Trägermaterial absorbiert, das Antikörper nach Anspruch 20 trägt, und anschließend das antigene Material davon eluiert.

**25.** Pharmazeutisches Präparat, dadurch **gekennzeichnet,** daß es ein antigenes Material nach einem der Ansprüche 1 bis 7, zusammen mit einem physiologisch annehmbaren Diluenten oder Träger umfaßt.

**26.** Antigenes Material nach einem der Ansprüche 1 bis 7 in Form eines Impfstoffs, geeignet zur Verwendung bei der Impfung gegen Malaria.

**27.** Antigenes Material nach einem der Ansprüche 1 bis 7 zur therapeutischen Anwendung.

FIG.1

FIG. 3

FIG. 2

FIG. 4

|  |  |  |  |  |  |  |  |  | (1)<br>GAATTC | CGC<br>Arg<br>[1] | AAG<br>Lys |
|---|---|---|---|---|---|---|---|---|---|---|---|
| TTT<br>Phe | ATA<br>Ile | TTT<br>Phe | GGA<br>Gly | GGT<br>Gly | GTT<br>Val | GGT<br>Gly | TTA<br>Leu | GTA<br>Val | TTA<br>Leu | TAC<br>Tyr |
| AAT<br>Asn | ACT<br>Thr | GAA<br>Glu | AAA<br>Lys | GGA<br>Gly | AGA<br>Arg | CAC<br>His | CCA<br>Pro | TTC<br>Phe | AAA<br>Lys<br>(100) | ATA<br>Ile |
| GGA<br>Gly | TCA<br>Ser | AGC<br>Ser | GAC<br>Asp | CCA<br>Pro | GCT<br>Ala | GAT<br>Asp | AAT<br>Asn | GCT<br>Ala | AAC<br>Asn | CCA<br>Pro |
| GAT<br>Asp | GCT<br>Ala | GAT<br>Asp | TCT<br>Ser | GAA<br>Glu | TCC<br>Ser | AAT<br>Asn | GGA<br>Gly | GAA<br>Glu | CCA<br>Pro | AAT<br>Asn |
| GCA<br>Ala | GAC<br>Asp | CCA<br>Pro | CAA<br>Gln<br>[50] | GTT<br>Val | ACA<br>Thr | GCT<br>Ala | CAA<br>Gln | GAT<br>Asp | GTT<br>Val<br>(200) | ACA<br>Thr |
| CCA<br>Pro | GAG<br>Glu | CAA<br>Gln | CCA<br>Pro | CAA<br>Gln | GGT<br>Gly | GAC<br>Asp | GAC<br>Asp | AAC<br>Asn | AAC<br>Asn | CTC<br>Leu |

GTA  AGT  GGC  CCT  GAA  CAC  TAAACAGCTGTAAACTTTTTTGTTAAT
Val  Ser  Gly  Pro  Glu  His
                                        (300)
GGGTTTTTTTGAAACACGTGAAAATAATTTTTATTTATGATTATATTATATATATTGCTATTT

TAAAAAAAAAAAAAAAAAAAAGGAATTC

FIG. 5a

36

<pre>
                                                                    (1)
                            GAATTCCTTTAATTTATTTAATATATTCAAA    ATG   AAA
                                                                Met   Lys
                                                                [1]

ATC   TTA   TCA   GTA   TTT   TTT   CTT   GCT   CTT   TTC   TTT
Ile   Leu   Ser   Val   Phe   Phe   Leu   Ala   Leu   Phe   Phe

ATC   ATT   TTC   AAT   AAA   GAA   TCC   TTA   GCC   GAA   AAA
Ile   Ile   Phe   Asn   Lys   Glu   Ser   Leu   Ala   Glu   Lys
                                                          (100)

ACA   AAC   AAA   GAA   ACT   GGA   AGT   GGT   GTT   AGC   AGC
Thr   Asn   Lys   Glu   Thr   Gly   Ser   Gly   Val   Ser   Ser

AAA   AAA   AAA   AAT   AAA   AAA   GGA   TCA   GGT   GAA   CCA
Lys   Lys   Lys   Asn   Lys   Lys   Gly   Ser   Gly   Glu   Pro

TTA   ATA   GAT   GTA   CAC   GAT   TTA   ATA   TCT   GAT   ATG
Leu   Ile   Asp   Val   His   Asp   Leu   Ile   Ser   Asp   Met
                        [50]                             (200)
ATC   AAA   AAA   GAA   GAA   GAA   CTT   GTT   GAA   GTT   AAC
Ile   Lys   Lys   Glu   Glu   Glu   Leu   Val   Glu   Val   Asn

AAA   AGA   AAA   TCC   AAA   TAT   AAA   CTT   GCC   ACT   TCA
Lys   Arg   Lys   Ser   Lys   Tyr   Lys   Leu   Ala   Thr   Ser

GTA   CTT   GCA   GGT   TTA   TTA   GGT   GTA   GTA   TCC   ACC
Val   Leu   Ala   Gly   Leu   Leu   Gly   Val   Val   Ser   Thr
                                                      (300)

GTA   TTA   TTA   GGA   GGT   GTT   GGT   TTA   GTA   TTA   TAC
Val   Leu   Leu   Gly   Gly   Val   Gly   Leu   Val   Leu   Tyr
                                                      [100]

AAT   ACT   GAA   AAA   GGA   AGA   CAC   CCA   TTC   AAA   ATA
Asn   Thr   Glu   Lys   Gly   Arg   His   Pro   Phe   Lys   Ile

GGA   TCA   AGC   GAC   CCA   GCT   GAT   AAT   GCT   AAC   CCA
Gly   Ser   Ser   Asp   Pro   Ala   Asp   Asn   Ala   Asn   Pro
                                                          (400)
GAT   GCT   GAT   TCT   GAA   TCC   AAT   GGA   GAA   CCA   AAT
Asp   Ala   Asp   Ser   Glu   Ser   Asn   Gly   Glu   Pro   Asn

GCA   GAC   CCA   CAA   GTT   ACA   GCT   CAA   GAT   GTT   ACA
Ala   Asp   Pro   Gln   Val   Thr   Ala   Gln   Asp   Val   Thr

CCA   GAG   CAA   CCA   CAA   GGT   GAC   GAC   AAC   AAC   CTC
Pro   Glu   Gln   Pro   Gln   Gly   Asp   Asp   Asn   Asn   Leu
                        [150]                       (500)
GTA   AGT   GGC   CCT   GAA   CAC   TAAACAGCTGTAAACTTTTTTGTTAAT
Val   Ser   Gly   Pro   Glu   His
</pre>

GGGTTTTTTTGAAACACGTGAAAATAATTTTTATTTATGATTATATTATATATATTGCTATTT

TAAAAAAAAAAAAAGGAATTC

## FIG. 5b

FIG. 6